# EUROPEAN PATENT SPECIFICATION

(11) **EP 1 309 857 B1**
(45) Date of publication and mention of the grant of the patent: **24.08.2005**
(21) Application number: 01944378.7
(22) Date of filing: 08.06.2001
(51) Int. Cl.: G01N 33/49

(54) **A METHOD FOR DETECTING A LIPOPROTEIN-ACUTE PHASE PROTEIN COMPLEX**
VERFAHREN ZUM NACHWEIS EINES LIPOPROTEIN-AKUTPHASEPROTEIN-KOMPLEXES
METHODE DE DETECTION D'UN COMPLEXE LIPOPROTEINE-PROTEINE DE PHASE AIGUE ET DE PREDICTION D'UN RISQUE ACCRU DE DEFAILLANCE DU SYSTEME OU DE MORTALITE

(30) Priority: 09.06.2000 US 591642
(43) Date of publication of application: 14.05.2003
(73) Proprietor: Biomerieux, Inc., Durham, NC 27712 (US)
(72) Inventor: FISCHER, Timothy, J., Tucson, AZ 85737 (US); DOWNEY, Colin, Liverpool, Merseyside L10 7NH (GB); NESHEIM, Mike, Kingston, Ontario K7K-6S4 (CA); SAMIS, John, A., Kingston, Ontario K7L 2S3 (CA); TEJIDOR, Liliana, Raleigh, NC 27615 (US); TOH, Cheng, Hock, Liverpool, Merseyside L25 6EQ (GB); WALKER, John, B., Kingston, Ontario K7M 5C6 (CA)
(74) Representative: Froud, Clive
(86) International application number: PCT/US2001/018611
(87) International publication number: WO 2001/096864

(56) References cited:
- WO-A-96/41291
- DOWNEY, C. ET AL: "Early identification and prognostic implications in disseminated intravascular coagulation through transmittance waveform analysis" THROMBOSIS AND HAEMOSTASIS, vol. 80, no. 1, July 1998 (1998-07), pages 65-69, XP001098607
- BHAKDI, S. ET AL: "Complement and atherogenesis: binding of CRP to degraded, nonoxidized LDL enhances complement activation" ARTHERIOSCLEROSIS, THROMBOSIS AND VASULAR BIOLOGY, vol. 19, no. 10, October 1999 (1999-10), pages 2348-2354, XP001098610
- TOH, C. H. ET AL: "THE MECHANISM UNDERLYING THE ATYPICAL CLOT WAVEFORM PROFILE OF DIC IS THROMBIN-INDEPENDENT BUT CALCIUM-DEPENDENT" ABSTRACT FORM EUROPEAN HAEMATOLOGY ASSOCIATION, 25 June 2000 (2000-06-25), page 1 XP002936645

## Description

### BACKGROUND OF THE INVENTION

Blood clots are the end product of a complex chain reaction where proteins form an enzyme cascade acting as a biologic amplification system. This system enables relatively few molecules of initiator products to induce sequential activation of a series of inactive proteins, known as factors, culminating in the production of the fibrin clot. Mathematical models of the kinetics of the cascade's pathways have been previously proposed.

Thrombosis and hemostasis testing is the in vitro study of the ability of blood to form clots and to break clots in vivo. Coagulation (hemostasis) assays began as manual methods where clot formation was observed in a test tube either by tilting the tube or removing fibrin strands by a wire loop. The goal was to determine if a patient's blood sample would clot after certain materials were added. It was later determined that the amount of time from initiation of the reaction to the point of clot formation in vitro is related to congenital disorders, acquired disorders, and therapeutic monitoring. In order to remove the inherent variability associated with the subjective endpoint determinations of manual techniques, instrumentation has been developed to measure *clot time,* based on (1) electromechanical properties, (2) clot elasticity, (3) light scattering, (4) fibrin adhesion, and (5) impedance. For light scattering methods, data is gathered that represents the transmission of light through the specimen as a function of time (an *optical time-dependent measurement profile*).

Two assays, the PT and APTT, are widely used to screen for abnormalities in the coagulation system, although several other screening assays can be used, e.g. protein C, fibrinogen, protein S and/or thrombin time. If screening assays show an abnormal result, one or several additional tests are needed to isolate the exact source of the abnormality. The PT and APTT assays rely primarily upon measurement of time required for clot time, although some variations of the PT also use the amplitude of the change in optical signal in estimating fibrinogen concentration.

Blood coagulation is affected by administration of drugs, in addition to the vast array of internal factors and proteins that normally influence clot formation. For example, heparin is a widely-used therapeutic drug that is used to prevent thrombosis following surgery or under other conditions, or is used to combat existing thrombosis. The administration of heparin is typically monitored using the APTT assay, which gives a prolonged clot time in the presence of heparin. Clot times for PT assays are affected to a much smaller degree. Since a number of other plasma abnormalities may also cause prolonged APTT results, the ability to discriminate between these effectors from screening assay results may be clinically significant.

The present invention was conceived of and developed for predicting haemostatic dysfunction in a sample based on one or more time-dependent measurement profiles, such as optical time-dependent measurement profiles. In addition, the present invention is directed to predicting the presence of Disseminated Intravascular Coagulation in a patient based on a time-dependent profile, such as an optical transmission profile, from an assay run on the patient's blood or plasma sample.

### SUMMARY OF THE INVENTION

The present invention is directed to a method for detecting a precipitate in a test sample in the absence of clot formation. The method includes providing a test sample and adding thereto a reagent, the reagent alone or in combination with additional reagents causing the formation of a precipitate. The reagent preferably comprises a metal divalent cation and optionally includes a clot inhibiting substance. The detection of the precipitate can be qualitative or quantitative, and the precipitate can be detected such as by a clotting assay, a latex agglutination or gold sol assay, an immunoassay such as an ELISA, or other suitable method that would allow for detection and/or quantitation of the precipitate. The formation of the precipitate can be detected as an endpoint value, or kinetically. This precipitate detection allows for predicting Haemostatic Dysfunction in patients. The present invention is useful for predicting Haemostatic Dysfunction that can lead to bleeding or thrombosis, or specifically to Disseminated Intravascular Coagulation (DIC).

More particularly, the present invention is directed to a method comprising adding a reagent to a test sample having at least a component of a blood sample from a patient, measuring the formation of a precipitate due to the reaction of the test sample and the reagent, over time so as to derive a time-dependent measurement profile, the reagent capable of forming a precipitate in the test sample without causing substantial fibrin polymerization.

The invention is also directed to a method for determining whether or not a patient has haemostatic dysfunction, comprising obtaining a blood sample from a patient, obtaining plasma from said blood sample, adding a reagent capable of inducing the formation of a precipitate in patients with haemostatic dysfunction without causing any substantial fibrin polymerization, taking one or more measurements of a parameter of the sample wherein changes in the sample parameter are capable of correlation to precipitate formation if present, and determining that a patient has haemostatic dysfunction if precipitate formation is detected.

The present invention is also directed to a method for determining in a patient sample the presence of a complex of proteins comprising at least one of a 300 kDa protein, serum amyloid A and C-reactive protein, comprising obtaining a test sample from a patient, adding an alcohol, a clot inhibitor, and a metal cation, wherein a precipitate is formed which comprises a complex of proteins including at least one of a 300 kDa protein, serum amyloid A and C-reactive protein.

The invention is also directed to a method comprising adding a coagulation reagent to an aliquot of a test sample from a patient, monitoring the formation of fibrin over time in said test sample by measuring a parameter of the test sample which changes over time due to addition of the coagulation reagent, determine a rate of change, if any, of said parameter in a period of time prior to formation of fibrin polymerization in said test sample, if the determined rate of change is beyond a predetermined threshold, then with a second aliquot of the patient test sample, add thereto a reagent that induces the formation of a precipitate in the absence of fibrin polymerization, measuring the formation of the precipitate over time, and determining the possibility or probability of haemostatic dysfunction based on the measurement of the precipitate.

The invention is also directed to a method for monitoring an inflammatory condition in a patient, comprising adding a reagent to a patient test sample, the reagent capable of causing precipitate formation in some patient test samples without causing fibrin polymerization, measuring a parameter of the test sample over time which is indicative of said precipitate formation, determining the slope of the changing parameter, repeating the above steps at a later date or time, wherein an increase or decrease in the slope at the later date or time is indicative of progression or regression, respectively, of the inflammatory condition.

The invention is further directed to a method for diagnosing and treating patients with haemostaic dysfunction, comprising adding a reagent to a test sample that causes precipitate formation without causing fibrin polymerization, taking measurements over time of a parameter of the test sample that changes due to the formation of the precipitate, determining the rate of change of said parameter, determining that a patient has haemostatic dysfunction if said rate of change is beyond a predetermined limit; intervening with treatment for said haemostatic dysfunction if said rate of change is beyond the predetermined limit.

The invention also is directed to a method comprising adding a reagent to a patient sample capable of causing formation of a precipitate in said sample, monitoring a changing parameter of said sample over time, said parameter indicative of said precipitate formation, determining the rate of change of said parameter or whether said parameter exceeds a predetermined limit at a predetermined time, repeating the above steps at least once, each time at a different plasma/reagent ratios, measuring the maximum, average and/or standard deviation for the measurements; and determining haemostatic dysfunction based on the maximum, average and/or standard deviation measurements.

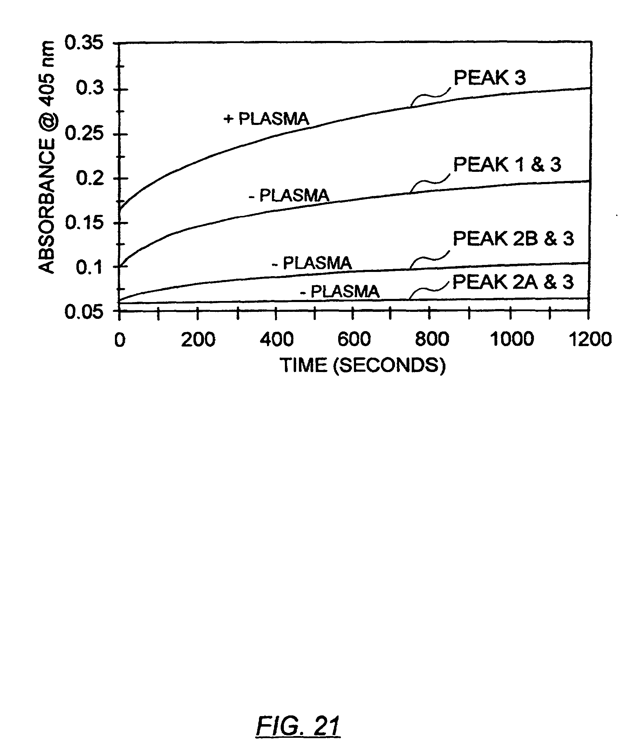

The present invention is further directed to an immunoassay comprising providing a ligand capable of binding to C-reactive protein or the 300 kDa protein in lane 5 of Fig. 21, adding said ligand to a test sample from a patient and allowing binding of said ligand to C-reactive protein or said 300 kDa protein in said test sample, detecting the presence and or amount of C-reactive protein or said 300 kDa protein in said sample, and diagnosing haemostatic dysfunction in the patient due to the detection and/or amount of C-reactive protein or said 300 kDa protein detected.

The invention further relates to a method for testing the efficacy of a new drug on a human or animal subject with an inflammatory condition and/or haemostatic dysfunction, comprising adding a reagent to a patient test sample, said reagent capable of causing precipitate formation in some subject test samples without causing fibrin polymerization, measuring a parameter of said test sample over time which is indicative of said precipitate formation, determining the slope of said changing parameter and/or the value of said parameter at a predetermined time, administering a drug to said animal or human subject, repeating the above steps at a later date or time, wherein an increase or decrease in said slope or value at said later date or time is indicative of the efficacy of said drug.

### BRIEF DESCRIPTION OF THE DRAWINGS

**Figures 1A and 1B** illustrate transmittance waveforms on the APTT assay with (**A**) showing a normal appearance, and (**B**) showing a biphasic appearance.Clot time is indicated by an arrow.
**Figure 2** illustrates transmittance levels at 25 seconds in relation to diagnosis in 54 patients with bi-phasic waveform abnormalities. The horizontal dotted line represents the normal transmittance level.
**Figure 3** illustrates serial transmittance levels (**A**)) and waveforms on day 1 (**B**), day 4 (**C**), and day 6 (**D**) on a patient who developed DIC following sepsis and recovered.
**Figure 4** illustrates serial transmittance levels (**A**) and waveforms on day 2 **(B),** day 5 (**C**), and day 10 (**D**)on a patient who developed DIC following trauma and died.
**Figure 5** illustrates ROC plots for the prediction of DIC transmittance at 25 seconds (TR25), APTT clot time, and slope_1 (the slope up to the initiation of clot formation).
**Figure 6** shows a histogram for DIC, normal and abnormal/non-DIC populations for TR25.
**Figure 7** shows a histogram for DIC, normal and abnormal/non-DIC populations for Slope_1.
**Figure 8** shows group distributions for slope_11.
**Figure 9** shows partial subpopulations of the data shown in **Figure 8**.
**Figure 10** shows group distributions for TR25.
**Figure 11** shows partial subpopulations of the data shown in **Figure 10**.
**Figure 12** is an optical transmission profile for an APTT assay using Platelin™.
**Figure 13** is an optical transmission profile for the PT assay using Recombiplast™.
**Figure 14** is an optical transmission profile for the PT assay using Thromborel S™.
**Figure 15** is a standard curve for ELISA of CRP.
**Figure 16** is a graph showing the time course of turbidity in a sample upon adding Ca²⁺ and PPACK compared to samples of normal and patient plasmas mixed in the various proportions indicated to the right. HBS/1 mM citrate was the diluent.
**Figure 17** is a graph showing the relationship between maximum turbidity change and amount of patient plasma in a sample.
**Figure 18** shows the results of anion exchange chromatography of material recovery after fractionation of patient plasma. Peaks of interest are indicated.
**Figures 19** shows non-reduced (**A**) and reduced (**B**) SDS-PAGE of various fractions of patient plasma.
**Figure 20** shows immunoblots of CRP in normal (**A** and **B**) and DIC plasma (**C**). In (**A**) and (**B**) lanes are labelled with the patient number; (**C**) is labeled with the ng amount of CRP loaded.
**Figure 21** illustrates the turbidity change upon adding divalent calcium to materials obtained upon Q-sepharose chromatography in the absence of plasma (except top curve).
**Figure 22** shows the response to increasing calcium concentrations in optical transmission profiles. Profiles are shown for two normal patients (**A, B**) and two patients with DIC (**C, D**).
**Figure 23** shows optical transmission profiles for calcium chloride alone (**B**) or in combination with APTT reagent (**A**). Numbers indicate patient ID numbers.
**Figure 24** is a calibration curve with heparin;
**Figure 25** shows CRP levels in 56 ITU patients plotted against transmittance at 18 seconds.
**Figure 26** shows more samples with CRP and decrease in transmittance at 18 seconds (10000- TR18).
**Figure 27** depicts a reconstitution experiment showing the effect on turbidity of combining VLDL and CRP (Peak 3), compared to VLDL alone. The starting concentration of VLDL for this experiment was 0.326 mg/mL.
**Figure 28** depicts a reconstitution experiment showing the effect on turbidity of combining IDL and CRP (Peak 3) compared to IDL alone. The starting concentration of IDL for this experiment was 0.06797 mg/mL.
**Figure 29** depicts a reconstitution experiment showing the effect on turbidity of combining LDL and CRP compared to LDL alone and CRP (Peak 3) alone. The starting concentration of LDL for this experiment was 0.354 mg/mL.
**Figure 30** depicts a reconstitution experiment showing the effect on turbidity of combining HDL and CRP (Peak 3) as compared to HDL alone. The starting concentration of HDL for this experiment was 1.564 mg/mL.
**Figure 31** is a ROC plot of sensitivity vs. specificity.
**Figure 32** is an immunoblot for apo(B)-100. Lane 1 is protein isolated from normal human plasma, lanes 2-5 are protein samples isolated from DIC patient plasma, and lanes 6-9 are calcium precipitates of protein samples from the same DIC patients in lanes 2-5. The monoclonal apo(B)-100 antibody was used at a 1/5000 dilution. Proteins were visualized with ECL reagents.
**Figure 33** is an SDS-PAGE gel of calcium precipitates from 4 DIC patients electrophoresed under reducing (lanes 1-4) or non-reducing (lanes 5-8) conditions. Approximately 5 µg of protein were loaded from patient #1 (lanes 1 and 5), patient # 2 (lanes 2 and 6), patient #3 (lanes 3 and 7), and patient #5 (lanes 4 and 8). After electrophoresis, the gel was stained with Coomassie Blue, destained, and dried.
**Figure 34** is an illustration of peaks 1 and 3 recovered from a Q-Sepharose column of washed calcium precipitate.
**Figure 35** is a graph depicting the turbidity changes associated with the addition of excess CRP and Ca⁺⁺ to isolated lipoproteins from normal plasma.
**Figure 36** is a graph depicting the quantitation of the interaction between CRP and VLDL. Recombinant CRP and normal VLDL were mixed at various concentrations in buffer and maximum turbidity changes were then recorded after adding Ca²⁺. The VLDL concentrations (measured as cholesterol) were: 0.030 mM (squares), 0.065 mM (triangles), 0.10 mM (diamonds), and 0.15 mM (circles). The lines are regression lines.
**Figure 37** is a graph depicting the quantitation of the interaction between CRP and VLDL. Recombinant CRP and normal VLDL were mixed at various concentrations in lipoprotein deficient plasma and maximum turbidity changes were then recorded after adding Ca²⁺. The VLDL concentrations (measured as cholesterol) were: 0.030 mM (squares), 0.065 mM (triangles), 0.10 mM (diamonds), and 0.15 mM (circles). The lines are regression lines.
**Figure 38** is a graph depicting the calcium concentration dependence of formation of the VLDL/CRP complex. Complex formation is half maximal at 5.0 mM calcium.
**Figure 39** is a graph depicting the turbidity changes associated with varying concentrations of VLDL in the presence of excess CRP in buffer and in lipoprotein-deficient plasma.
**Figure 40** is a graph depicting the inhibition of VLDL/CRP complex formation by EACA. The IC₅₀ for inhibition by EACA is 2.1 mM.
**Figure 41** is a graph depicting turbidity change versus varying CRP concentration.
**Figure 42** is a graph depicting correlations between the level of CRP in complex with VLDL and the turbidity change upon recalcification of patient plasma samples. The total concentration of CRP and VLDL (cholesterol) in 15 patient plasmas were measured. The level of CRP in complex was calculated, using the parameters for complex formation measured in lipoprotein depleted normal plasma, supplemented with normal VLDL and recombinant CRP. The absorbance change at 405 nm (turbidity) was measured 20 minutes after adding CaCl₂ and the thrombin inhibitor PPACK to the samples.
**Figure 43** is a graph depicting the correlation between the VLDL levels and turbidity changes upon recalcification of patient plasma versus varying VLDL concentration.
**Figure 44** is a graph depicting MDA waveforms for normal, bi-phasic, and bi-phasic/thrombin inhibitor samples.
**Figure 45** is non-reducing SDS-PAGE gel of isolated precipitate before and after anion exchange chromatography. Lanes 1-3 were loaded with the starting material, peak 1, and peak 3, respectively.
**Figure 46** are non-reducing SDS-PAGE gels that were immunoblotted and probed with either anti-APO(B) (**A**), anti-CRP (**B**), or anti-SAA (**C**) antibody. The blots represent the analysis of isolated precipitate before and after anion exchange chromatography. Lanes' 1-3 were loaded with the starting material, peak 1, and peak 3, respectively.
**Figure 47** is a graph depicting the turbidity changes associated with the a mixture of peaks 1 and 3 isolated from anion exchange chromatography.
**Figure 48** is a graph showing the time course of turbidity changes after adding Ca⁺⁺ to mixtures of normal plasma and the plasma of a patient with a biphasic waveform. The values at the right are volumes of patient plasma in a total of 50 µL.
**Figure 49** is a graph depicting a standard curve assay of the change in turbidity associated with varying amounts of patient plasma added. 1 mL of patient plasma = 1 unit of activity.
**Figure 50** is a graph depicting the effect of EACA on Ca⁺⁺-dependent turbidity changes associated with VLDL and CRP.

### DESCRIPTION OF THE PREFERRED EMBODIMENTS

In the present invention, not only can a particular abnormality (Haemostatic Dysfunction) be detected, but in addition the progression of the disease can be monitored in a single patient. More particularly, system failure and/or mortality can be predicted. Haemostatic Dysfunction, as used herein, is a condition evidenced by the formation of a precipitate (prior to or in the absence of clot formation), depending upon the reagent used).

Disseminated intravascular coagulation (DIC - a type of Haemostatic Dysfunction) prognosis has been hampered by the lack of an early, useful and rapidly available diagnostic marker. The invention has been found to be not only useful as an early diagnostic and single monitoring marker of DIC, but in addition the quantifiable and standardizable changes also allow for prognostic applicability in clinical management.

Disseminated intravascular coagulation (DIC) is a secondary response to a pre-existing pathology whereby the haemostatic response becomes perturbed and disseminated as opposed to the focused events of normal haemostasis. Despite improvements both in the intensive care management of patients and in our basic knowledge of haemostatic mechanisms in DIC, survival in this patient group is still very discouraging. Fundamental to the management of this complication is the implementation of aggressive therapy directed at forestalling or eradicating the primary pathology as the source of the initiating stimulus. However, in practical terms, the problem remains one of early identification of DIC to facilitate immediate and appropriate intervention. Although the technological armory available to the clinical investigator has expanded enormously, the pace of acute DIC precludes most of the more specific tests and reliance is still placed on traditional screening tests such as the prothrombin (PT), activated partial thromboplastin time (APTT) and platelet count. These tests lack specificity on an individual basis and are only useful in DIC if they lead on to further determinations of fibrinogen and fibrin breakdown products/D-dimers. However, changes in these parameters may not occur all at the same time and as such, serial testing is often needed which inevitably leads to a delay in diagnosis and clinically useful intervention.

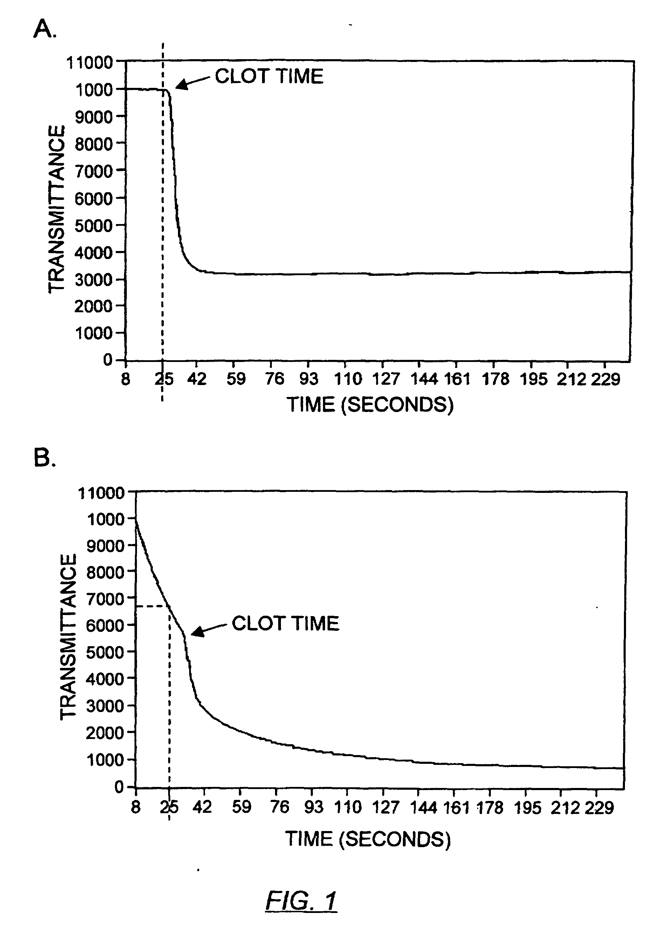

The normal sigmoidal appearance from an APTT transmittance waveform (TW) changes to a "bi-phasic" appearance in DIC patients. This represents a loss in the plateau of a normal APTT-TW, with development of an initial low gradient slope followed by a much steeper slope (Figures 1a and b). In addition, this bi-phasic pattern can be seen even when the APTT clotting time result is normal.

Freshly collected blood samples that required a PT or an APTT were analyzed prospectively over a two week working period. These were in 0.105 M tri-sodium citrate in the ratio of 1 part anticoagulant to 9 parts whole blood and the platelet-poor plasma was analyzed on the MDA (Multichannel Discrete Analyzer) 180, an automated analyzer for performing clinical laboratory coagulation assays using an optical detection system (Organon Teknika Corporation, Durham, NC, USA). In addition, to deriving the clot times for both PT (normal 11.2-15s) using MDA Simplastin LS™ and APTT (normal 23-35s) using MDA Platelin LS™ with 0.025M calcium chloride (Organon Teknika Corporation, USA), an analysis of the TW for the APTT was performed on each occasion at a wavelength of 580nm. To quantitate the visual profile, the amount of light transmittance at 25 seconds was recorded. A normal waveform has a light transmittance of 100% that is represented on the analyzer and in Figure 1a without the decimal point as 10000. As such, a bi-phasic change will have a reduced light transmittance of less than 10000. As can be seen in **Figure 1B,** decreasing levels of light transmittance prior to clot formation correlate directly with increasing steepness of the bi-phasic slope. The recording of the light transmittance at 25 seconds also allows for standardization between patients and within the same patient with time. If the minimum level of light transmittance for each sample were to be used instead, this would be affected by variations in the clot time of the APTT and would therefore not be ideal for comparisons.

To ensure that no cases of DIC were overlooked, the following criteria was followed. If (a) an abnormal bi-phasic TW was encountered, or (b) a specific DIC screen was requested, or (c) if there was a prolongation in either the PT or APTT in the absence of obvious anticoagulant therapy, a full DIC screen was performed. This would further include the thrombin time (TT) (normal 10.5-15.5 seconds), fibrinogen (Fgn)(normal 1.5-3.8 g/l) and estimation of D-dimer levels (normal < 0.5 mg/l) on the Nyocard D-Dimer (Nycomed Pharma AS, Oslo, Norway). Platelet counts (Plt) (normal 150-400 10⁹/l) performed on an EDTA sample at the same time were recorded. In addition, clinical details were fully elucidated on any patient with a bi-phasic TW or coagulation abnormalities consistent with DIC.

The diagnosis of DIC was strictly defined in the context of both laboratory and clinical findings of at least 2 abnormalities in the screening tests (increased PT, increased APTT, reduced Fgn, increased TT or reduced Plt) plus the finding of an elevated D-dimer level (>0.5 mg/l) in association with a primary condition recognized in the pathogenesis of DIC. Serial screening tests were also available on those patients to chart progression and confirmation of the diagnosis of DIC as was direct clinical assessment and management. For statistical analysis, values for the sensitivity, specificity, positive and negative prediction of the APTT-TW for the diagnosis of DIC were calculated employing a two-by-two table. 95% confidence intervals (CI) were calculated by the exact binomial method.

A total of 1,470 samples were analyzed. These were from 747 patients. 174 samples (11.9%) from 54 patients had the bi-phasic waveform change. 22 of these 54 patients had more than 3 sequential samples available for analysis. DIC was diagnosed in 41 patients with 30 of these requiring transfusion support with fresh frozen plasma, cryoprecipitate or platelets. The underlying clinical disorders as shown in **Table 1**.

**TABLE 1**

| Disorder | No |
|---|---|
| Infections | 17 |
| Trauma or recent major surgery | 16 |
| Malignancy | 2 |
| Hepatic Disease | 1 |
| Obstetric | 1 |
| Miscellaneous Additional Causes * | 4 |

| | |
|---|---|
| * Includes hypoxia, acidosis, Lithium overdosage and graft rejection | |

40 of the 41 patients with DIC had the bi-phasic TW. The one false negative result (DIC without a bi-phasic TW) occurred in a patient with pre-eclampsia (PET) where the single sample available for analysis showed a prolonged PT of 21.0s, APTT of 44.0s and raised D-dimers of 1.5mg/l. 5 other patients were identified in this study with PET and none had either DIC or a bi-phasic TW. Of the 14 patients with a bi-phasic TW which did not fulfil the criteria of DIC, all had some evidence of a coagulopathy with abnormalities in one or two of the screening tests. These abnormal results fell short of the criterion for DIC as defined above. 4 of these 14 patients had chronic liver disease with prolonged PT and mild thrornbocytopaenia. A further 2 patients had atrial fibrillation with isolated elevation of D-dimer levels only. The remaining 8 patients were on the ICU with multiple organ dysfunction arising from trauma or suspected infection but without the classical laboratory changes of DIC. These patient profiles were described in the ICU as consistent with the "systemic inflammatory response syndrome" (SIRS). Based on these figures, the bi-phasic TW has a 97.6% sensitivity for the diagnosis of DIC with a specificity of 98%. Use of an optical transmittance waveform was found to be helpful in detecting the biphasic waveform.

**TABLE 2**

| | Biphasic TW | Normal TW | Total |
|---|---|---|---|
| DIC Positive | 40 | 1 | 41 |
| DIC Negative | 14 | 692 | 706 |
| Total | 54 | 693 | 747 |
| Sensitivity 97.6% (Cl 85.6-99.99%), Specificity 98.0% (Cl 96.6-98.9%), Positive predictive value 74.0% (Cl 60.1-84.6%),Negative predictive value 99.9% (cl99.1-99-99%) | | | |

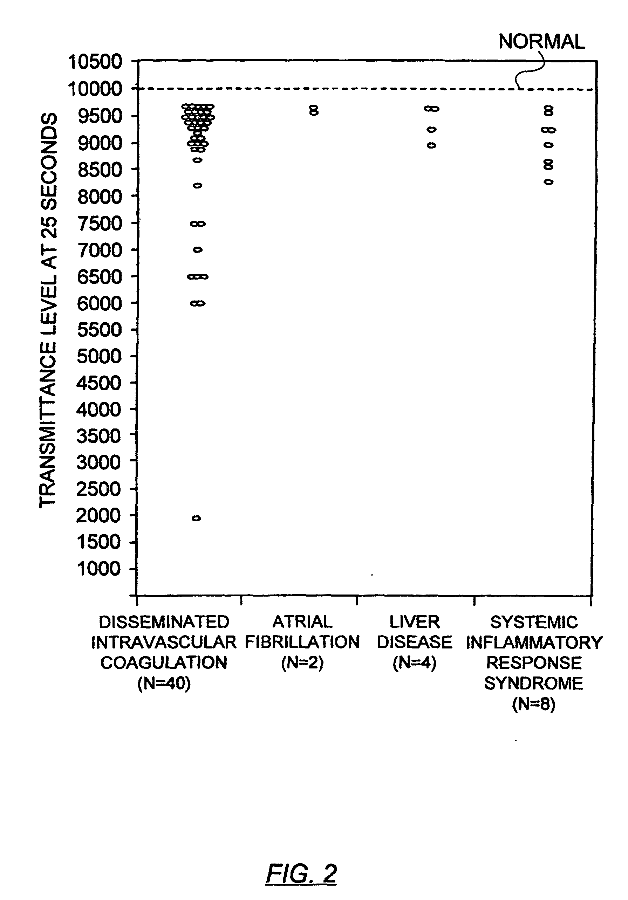

The positive predictive value of the test was 74%, which increased with increasing steepness of the bi-phasic slope and decreasing levels of light transmittance (**Table 2** and **Figure 2**). In the first two days of the study, there were 12 patients who had an abnormality in the clotting tests plus elevation of D-dimer levels. These were patients who were clinically recovering from DIC that occurred in the week preceding the study. This led to the impression that TW changes might correlate more closely with clinical events than the standard markers of DIC.

**TABLE 3**

| Day | Time | PT (11.2-15s) | APTT (23-35s) | TT (10.5-15.5s) | Fgn (1.5-3.8 g/l) | D-Dimer (<0.5 mg/l) | Pit (150-400x 10⁹/l | TW |
|---|---|---|---|---|---|---|---|---|
| 1 | 0923 | 14.7 | 32.9 | 12.0 | 4.7 | 0.00 | 193 | B* |
| 1 | 2022 | 20.8* | 38.6* | 12.4 | 5.7 | 6.00* | 61* | B* |
| 2 | 0920 | 18.0* | 33.0 | 13.0 | 5.2 | 2.00* | 66* | N |
| 3 | 1011 | 16.3* | 24.8 | 13.2 | 4.7 | 0.00 | 64* | N |
| PT=Prothrombin time, APTT=Activated Partial Thromboplastin Time, TT=Thrombin Time, Fgn=Fibrinogen, PTT=Platelet count, TW=Transmittance Waveform | | | | | | | | |

| | | | | | | | | |
|---|---|---|---|---|---|---|---|---|
| *Indicates abnormal changes, B=bi-phasic, N=normal | | | | | | | | |

The availability of more than 3 sequential samples in 22 patients allowed for further assessment. **Table 3** illustrates one such example with serial test results from a patient with *E. coli* septicaemia.

The appearance of a bi-phasic TW preceded changes in the standard tests for the diagnosis of DIC. It was only later in the day that the PT, APTT, Plt and D-dimer levels became abnormal and fulfilled the diagnostic criteria of DIC. Treatment with intravenous antibiotics led to clinical improvement by Day 2 with normalization of her TW in advance of the standard parameters of DIC. D-dimers and Plt were still respectively abnormal 24 and 48 hours later.

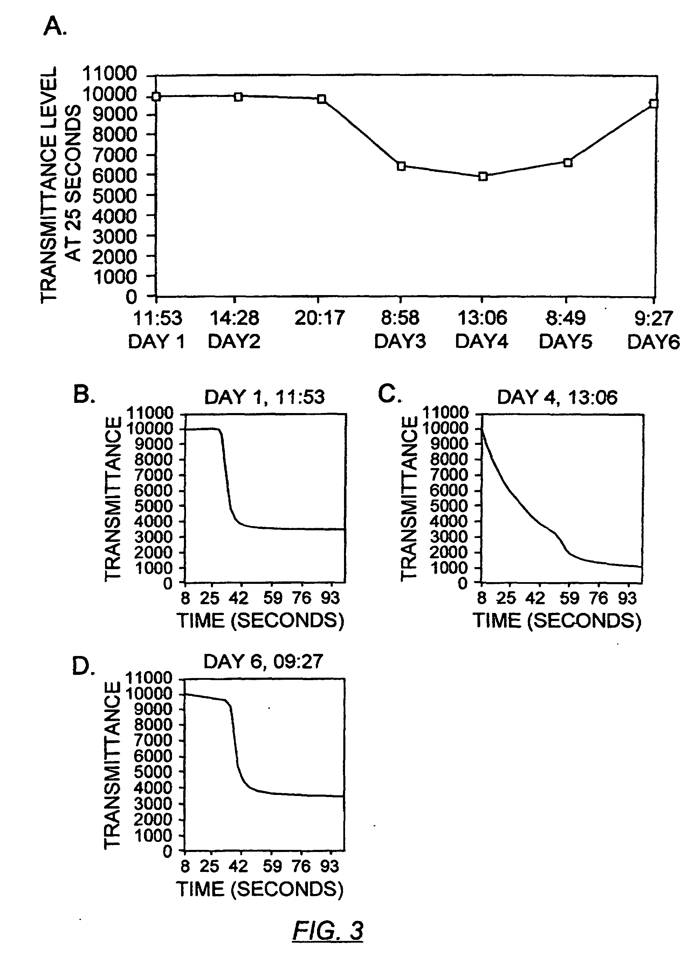

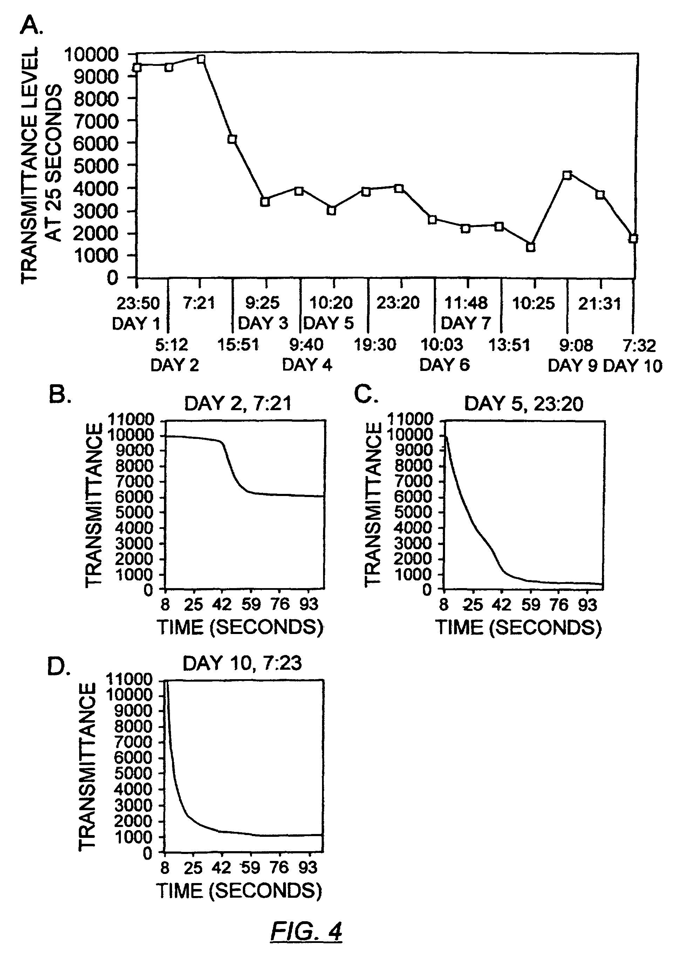

This correlation between clinical events and TW changes was seen in all the DIC patients where samples were available to chart the course of clinical events. As the TW changes were quantifiable and standardizable through recording of the transmittance level at 25 seconds, this analysis provided a handle in assessing prognostic applicability. Figure 3 illustrates the results of a patient who initially presented with peritonitis following bowel perforation. This was further complicated by gram negative septicaemia post-operatively with initial worsening of DIC followed by a gradual recovery after appropriate therapy. As DIC progressed initially, there was increasing steepness in the bi-phasic slope of the TW and a fall in the light transmittance level. A reversal of this heralded clinical recovery. Figure 4 illustrates the results of a patient who sustained severe internal and external injuries following a jet-ski accident. Although initially stabilized with blood product support, his condition deteriorated with continuing blood loss and development of fulminant DIC. The bi-phasic slope became increasingly steep with falls in transmittance level as the consequences of his injuries proved fatal.

As DIC can arise from a variety of primary disorders, the clinical and laboratory manifestations can be extremely variable not only from patient to patient but also in the same patient with time. There is therefore, a need for systems that are not only robust in their diagnosis but simple and rapid to perform. Although it has been shown that the bi-phasic TW appeared to be sensitive for Haemostatic Dysfunction (e.g. DIC) and was not seen in other selected patient groups with coagulation aberrations or influenced by either (i) pre-analytical variables, (ii) different silica-based APTT reagents, (iii) the use of thrombin as the initiator of the coagulation reaction or (iv) treatment in the form of heparin or plasma expanders, the robustness of this assay for DIC could only be addressed through a prospective study. This study has shown that the bi-phasic TW provides diagnostic accuracy in DIC with an overall sensitivity of 97.6% and specificity of 98%. In contrast, none of the standard parameters on an individual basis (i.e., PT, APTT, TT, Fgn, Plt, D-dimers) or even in combination, has ever reached the degree of sensitivity or specificity. The ready availability of TW data from the MDA-180 would also fulfil the criteria of simplicity and rapidity unlike the measurements of thrombin-antithrombin complexes or other markers that are dependent on ELISA technology. In addition, the advantages of TW analysis are that: (a) the bi-phasic TW change appears to be the single most useful correlate within an isolated sample for DIC and as such, reliance need no longer be placed on serial estimations of a battery of tests, and (b) the appearance or resolution of the bi-phasic TW can precede changes in the standard, traditional parameters monitored in DIC with strong, clear correlation to clinical events and outcome.

Although the bi-phasic TW was also seen in patients who did not have DIC per se as defined by the above criteria, the clinical conditions were associated with Haemostatic Dysfunction - namely activated coagulation prior to initiation of clot formation resulting in a biphasic waveform (for example in chronic liver disease or in the very ill patients on the Intensive Care Unit who had multiple organ dysfunction). It appears that bi-phasic TW is sensitive to non-overt or compensated DIC and that a transmittance level of less than 90% **(Figure 2)** or sequential falls in that level **(Figure 4),** reflects decompensation towards a more overt manifestation and potentially fulminant form of DIC. This line of explanation is supported by the observation of only a mild bi-phasic TW (transmittance level of about 95%) in 2 patients with atrial fibrillation; a condition that is associated with mild coagulation activation and elevated D-dimer levels. As no follow-up samples were available on these 2 patients whose clinical details were otherwise unremarkable, their bi-phasic TW could well have been transient. Nonetheless, these cases illustrate that the lower the level of light transmittance, the more likely the bi-phasic TW becomes predictive of Haemostatic Dysfunction, particularly DIC.

The observation of a normal TW in a patient with PET and DIC needs further exploration as the study did not selectively aim to examine any particular patient groups and only had a total of 6 patients with PET; the remaining 5 of which did not have DIC. One explanation which would be supported by other findings in this study is that the patient could have been recovering from PET and DIC at the time of the sample. There may already have been normalization in the bi-phasic TW in advance of the other parameters which were still abnormal and indicative of DIC. Another explanation is that the disturbed haemostatic process in PET is more localized and different from the DIC that arises from other conditions. Such patients respond dramatically to delivery of the fetus which suggests anatomical localization of the pathological process to the placenta despite standard laboratory clotting tests implying systemic evidence of the condition.

### Example:

Though analysis of the transmittance at a time of 25 seconds is helpful in predicting DIC, a second embodiment of the invention has been found that greatly improves sensitivity and specificity. It has been found that looking at transmittance at a particular time can result in detecting an artifact or other decrease in transmittance at that point, even though the waveform is not a bi-phasic waveform. For example, a temporary dip in transmittance at 25 seconds would cause such a patient sample to be flagged as bi-phasic, even if the waveform was normal or at least not bi-phasic. Also, if a patient sample had a particularly short clotting time, then if clot formation begins e.g. prior to 25 seconds (or whatever time is preselected), then the waveform could be flagged as biphasic, even though the real reason for decreased transmittance at 25 seconds is because clot formation has already begun/occurred.

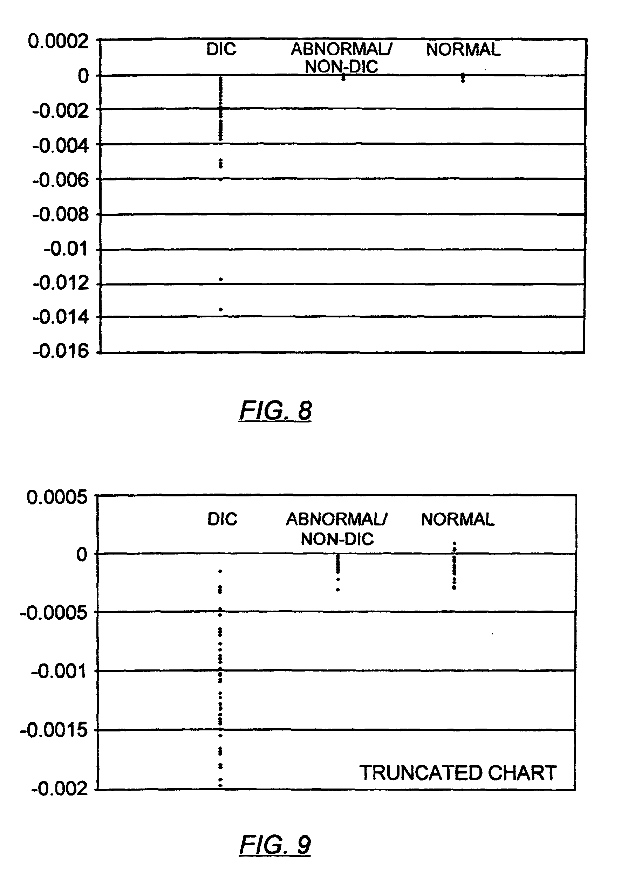

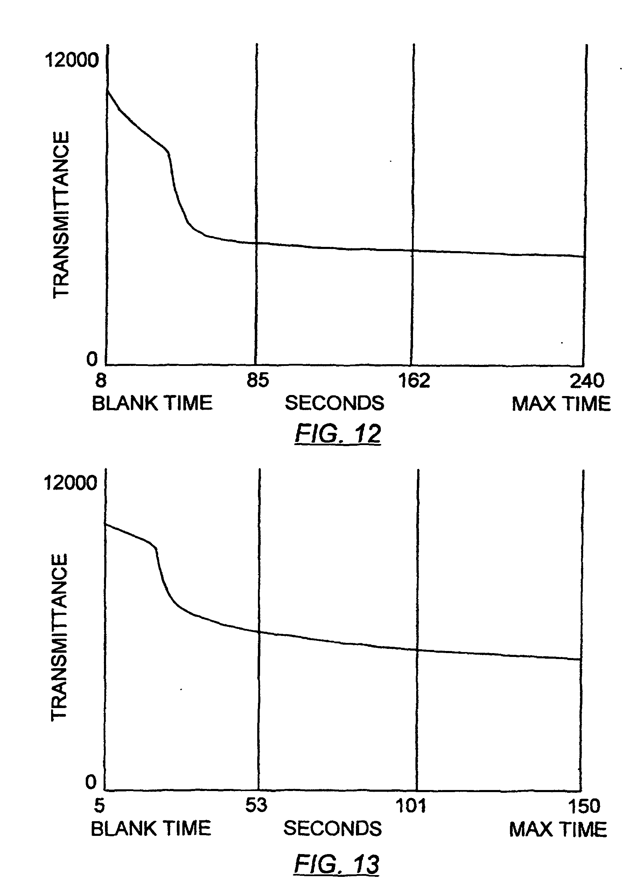

For this reason, it has been found that rather than analysis of transmittance at a particular time, it is desirable to calculate the slope of the waveform prior to initiation of clot formation. This calculation can involve determination of clot time followed by determination of waveform slope prior to clot time. In an additional embodiment, the slope (not transmittance) is determined prior to clot time or prior to a preselected time period, whichever is less. As can be seen in **Figure 11,** when transmittance is used for determining e.g. DIC, there is poor specificity and sensitivity. However, as can be seen in **Figure 9**, when slope prior to initiation of clot formation is used, specificity and sensitivity are greatly improved, and are better than standard tests used in the diagnosis of Haemostatic Dysfunction, such as DIC.

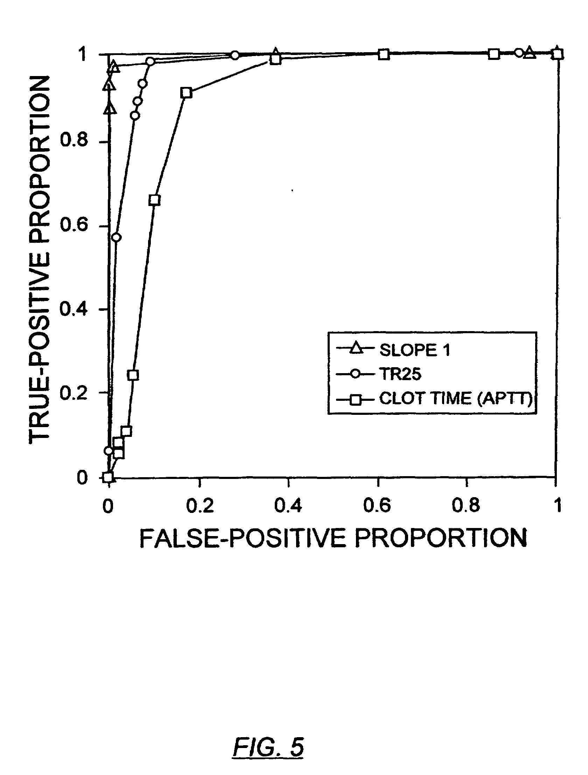

Additional testing was performed on three sets of patients. The first set consisted of 91 APTT assays run on samples from 51 different confirmed DIC patients. The second set of data consisted of 110 APTT assays run on samples from 81 different confirmed normal patients. The third set of data included 37 APTT assays run on 22 abnormal, non-DIC samples. **Figure 5** illustrates ROC plots for the prediction of DIC for three different parameters derived from the APTT assay using the combined data sets described: (1) transmittance at 25 seconds (TR25), (2) APTT clot time, and (3) slope 1 (the slope up to initiation of clot formation). Slope 1 exhibited the best predictive power, followed by TR25. It has also been shown that transmittance at 18 seconds has predictive value, particularly when the APTT clot time is less than 25 seconds. The "cutoffs" associated with the highest efficiency for the three parameters are listed in **Table 4**:

**Table 4**

| Parameter | Cutoff |
|---|---|
| TR25 | < 9700 |
| Clot Time | > 35 |
| Slope 1 | < -0.0003 |

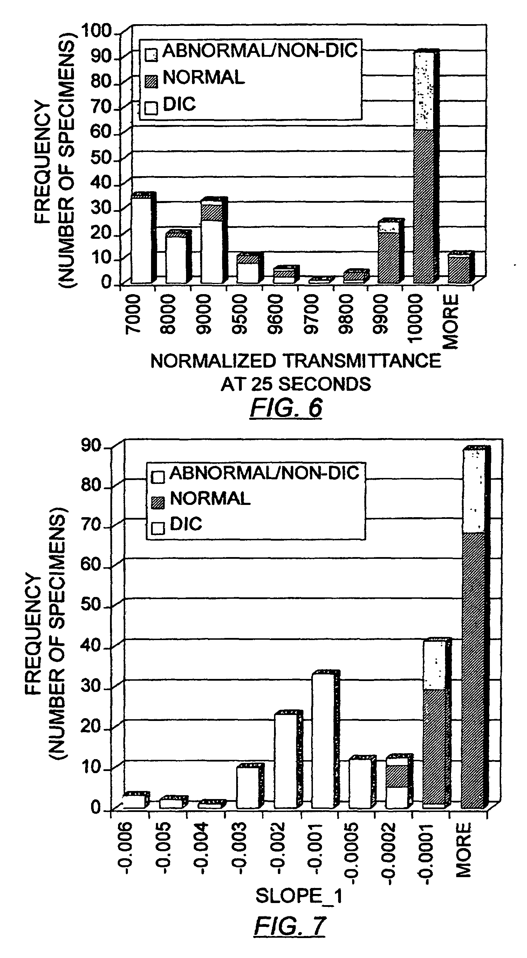

It should be noted that these cutoffs have shifted with the addition of the third set, and would likely shift again, depending on the sample populations. **Figures 6** and 7 show the histograms for the DIC, normal and abnormal/non-DIC populations for TR25 and slope 1 respectively. Tables 5 and 6 show the data for the histograms in Figures 6 and 7 respectively:

**TABLE 5**

| Bins | DIC | Normal | Abnormal/Non-DIC |
|---|---|---|---|
| | | | |
| -0.006 | 3 | 0 | 0 |
| -0.005 | 2 | 0 | 0 |
| -0.004 | 1 | 0 | 0 |
| -0.003 | 10 | 0 | 0 |
| -0.002 | 24 | 0 | 0 |
| -0.001 | 33 | 0 | 0 |
| -0.0005 | 12 | 0 | 0 |
| -0.0002 | 5 | 5 | 2 |
| -0.0001 | 1 | 37 | 13 |
| More | 0 | 68 | 22 |

**TABLE 6**

| *Bin* | DIC | Normal | Abnormal/Non-DIC |
|---|---|---|---|
| 7000 | 34 | 1 | 0 |
| 8000 | 18 | 2 | 0 |
| 9000 | 26 | 6 | 1 |
| 9500 | 8 | 3 | 0 |
| 9600 | 3 | 2 | 1 |
| 9700 | 1 | 0 | 0 |
| 9800 | 1 | 3 | 0 |
| 9900 | 0 | 21 | 4 |
| 10000 | 0 | 62 | 30 |
| More | 0 | 10 | 1 |

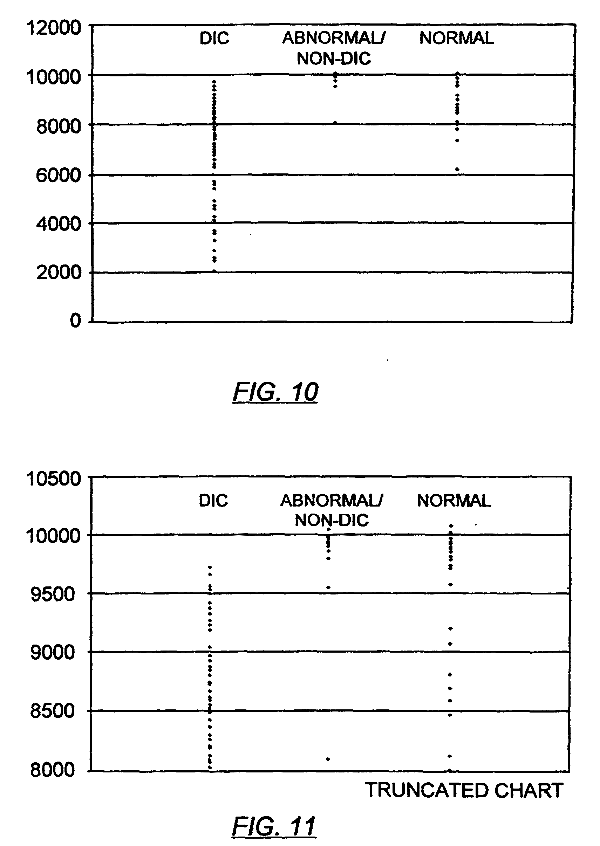

**Figures 8** and **10** show the group distributions for Slope 1 and TR25 respectively; and **Figures 9** and **11** show the group distributions for Slope 1 and TR25 respectively. **Figures 9** and **11** show partial subpopulations of the data shown in **Figures 8** and **10.**

When the prediction of Haemostatic Dysfunction is performed on an automated or semi-automated analyzer, the detected bi-phasic waveform can be flagged. In this way, the operator of the machine, or an individual interpreting the test results (e.g. a doctor or other medical practitioner) can be alerted to the existence of the biphasic waveform and the possibility/probability of Haemostatic Dysfunction such as DIC. The flag can be displayed on a monitor or printed out. A slope of less than about -0.0003 or less than about -0.0005 is the preferred cutoff for indicating a bi-phasic waveform. An increasing steepness in slope prior to clot formation correlates to disease progression.

The above examples show that waveform analysis on the APTT assay can identify characteristic bi-phasic patterns in patients with haemostatic dysfunction. In the majority of cases, this dysfunction could be labelled as DIC. This diagnostic waveform profile was seen in all APTT reagents tested, which were either silica or ellagaic acid-based. It has also been surprisingly found that a bi-phasic waveform can also be seen on PT assays with particular reagents, and that the bi-phasic waveform is likewise indicative of haemostatic dysfunction, primarily DIC.

Using samples that give bi-phasic APTT waveforms, the PT waveform profile was derived using PT reagents (thromboplastin), namely Recombiplast™ (Ortho), Thromborel™ (Dade-Behring) and Innovin™ (Dade-Behring). Both Recombiplast™ and Thromborel™ were particularly good at showing bi-phasic responses. Innovin™ was intermediate in its sensitivity. Using the transmittance level at 10 seconds into the PT reaction as the quantitative index, Recombiplast™ and Thromborel™ objectively showed lower levels of light transmittance than Innovin™. Thromborel™ can show a slight increase in initial light transmittance before the subsequent fall. This may be, in part, related to the relative opaqueness of Thromborel™.

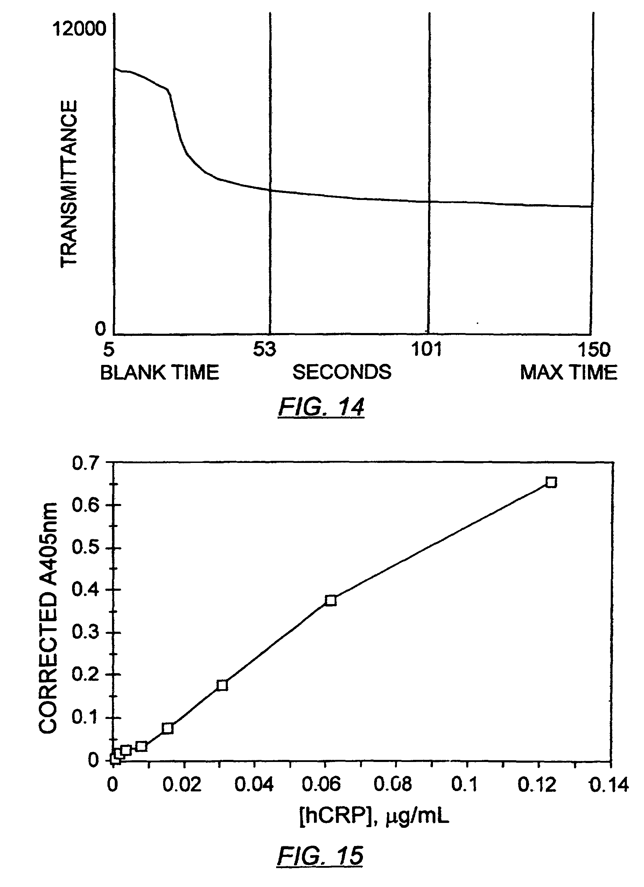

Further studies were performed comparing APTT profiles using Platelin™ and PT waveform profiles using Recombiplast™. Consecutive samples over a four week period from the intensive care unit were assessed. Visually, and on objective scores (comparing TL18 for APTT and TL10 for PT), the APTT profile was more sensitive to changes of haemostatic dysfunction and clinical progression than the PT profile. This relative sensitivity can be seen in the APTT profile of **Figure 12** (Platelin) compared to the PT profiles of **Figure 13** (Recombiplast) and **Figure 14** (Thromborel S). Invariably, at smaller changes in light transmittance, the APTT waveform detected abnormalites more easily than the PT waveform. Nonetheless, in severe degrees of haemostatic dysfunction, both bi-phasic profiles were concordant.

In a further embodiment of the invention, the time dependent measurement, such as an optical transmittance profile, can be performed substantially or entirely in the absence of clot formation. In this embodiment, a reagent is added which causes the formation of a precipitate, but in an environment where no fibrin is polymerized. The reagent can be any suitable reagent that will cause the formation of a precipitate in a sample from a patient with haemostatic dysfunction, such as DIC. As an example, divalent cations, preferably of the transition elements, and more preferably calcium, magnesium, manganese, iron or barium ions, can be added to a test sample. These ions cause activation of an atypical waveform that can serve as an indicator of haemostatic dysfunction. It is also possible to run this assay in the absence of a clotting reagent (APTT, PT, or otherwise). As part of the reagent that comprises the activator of the atypical waveform, or separately in another reagent, can also be provided a clot inhibitor. The clot inhibitor can be any suitable clot inhibitor such as hirudin, PPACK, heparin, antithrombin, I2581, etc. The formation of the atypical waveform can be monitored and/or recorded on an automated analyzer capable of detecting such a waveform, such as one that monitors changes in turbidity (e.g. by monitoring changes in optical transmittance).

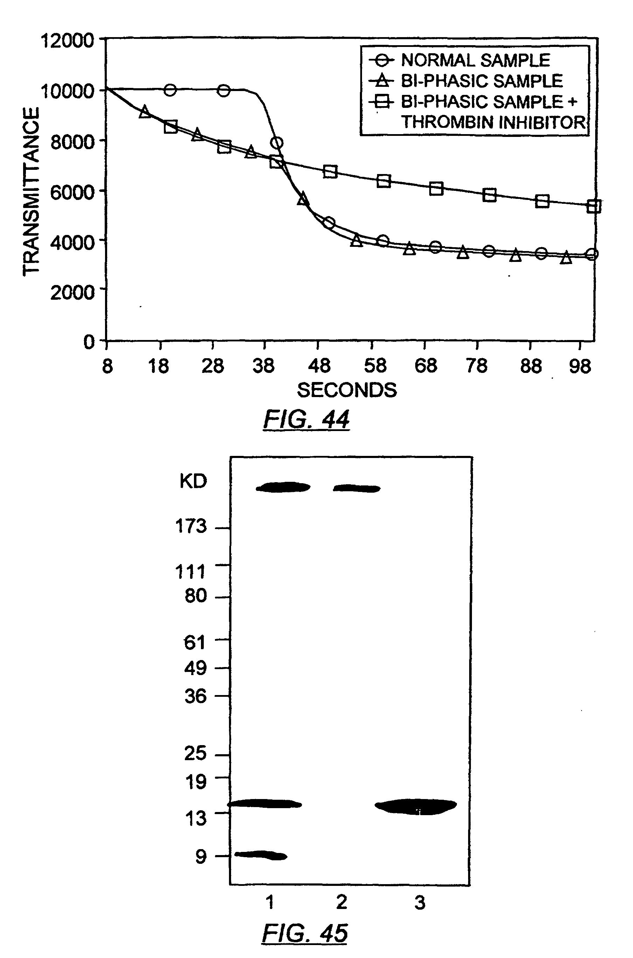

**Figure 44** is an illustration of two waveforms: waveform (triangles) is a test run on a sample using an APTT clotting reagent and resulting in an atypical (biphasic) waveform, whereas waveform (squares) is a test run on a sample where a clot inhibitor is used (along with a reagent, such as a metal divalent cation, which causes the formation of a precipitate in the sample). Waveform (squares) is exemplary of a waveform that can result in patients with haemostatic dysfunction where no clotting reagent is used and/or a clot inhibitor is added prior to deriving the time-dependent measurement profile. Generally speaking, the greater the slope of the waveform (the larger the drop in transmittance in the same period of time) due to the precipitate formation, the greater severity of the patient's haemostatic dysfunction. **Figure 15** is a standard curve for ELISA of CRP (CRP isolated from a patient used as the standard).

The precipitate formed in the present invention was isolated and characterized by means of chromatography and purification. Gel Filtration was performed as follows: A column (Hiprep Sephacryl S-300 High resolution - e.g. resolution of 10 to 1500 kDa) was used. The volume was 320 ml (d=26mm, 1=600mm), and the flow rate was 1.35 ml/min.

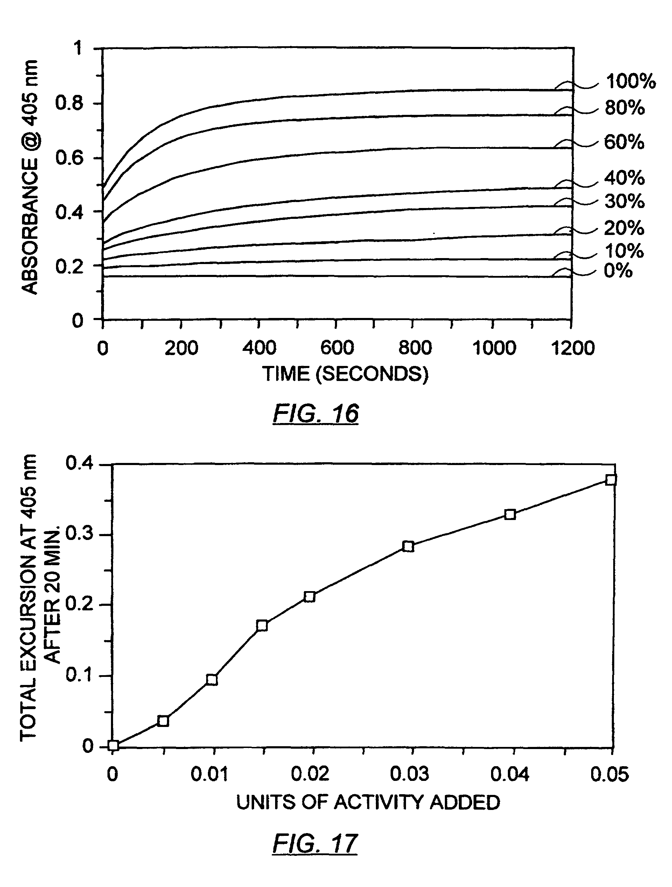

**Figure 16** is a graph showing the time course of turbidity in a sample upon adding a precipitate inducing agent (in this case divalent calcium) and a thrombin inhibitor (in this case PPACK) to mixtures of patient and normal plasmas. **Figure 17** is a graph showing the relationship between maximum turbidity change and amount of patient plasma in one sample. 0.05 units implies 100% patient plasma.

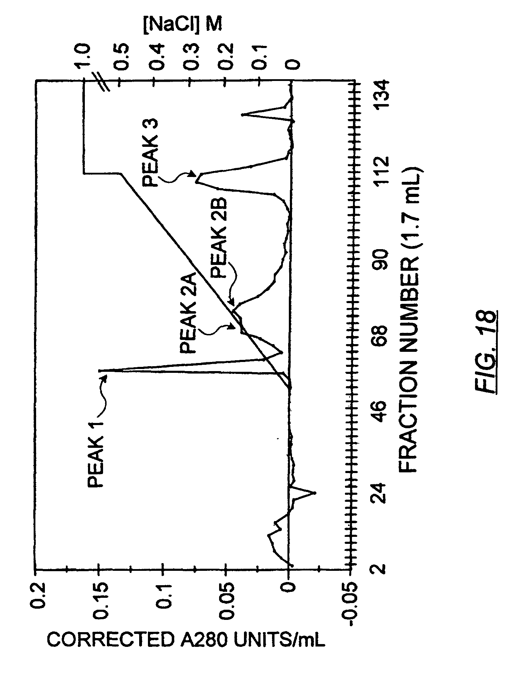

The steps used in the purification of components involved in the turbidity change in a patient's plasma were as follows: PPACK (10 µM) was added to patient plasma. Calcium chloride was added to 50mM, followed by 8 minutes of incubation, followed by the addition of ethanol to 5%. The sample was then centrifuged 10,500 x g for 15 minutes at 4 degrees Celsius. The pellet was then dissolved in HBS/1mM citrate/10 µM PPACK, followed by 35-70% (NH₄)₂SO₄ fractionation. Finally, an ion exchange chromatography was performed using a 5ml bed, 0.02-0.5M NaCl gradient and 50ml/side, to collect 2ml fractions. **Figure 18** shows the results of anion exchange chromatography (Q-sepharose) of material recovered after the 35-70% ammonium sulfate fractionation of patient plasma.

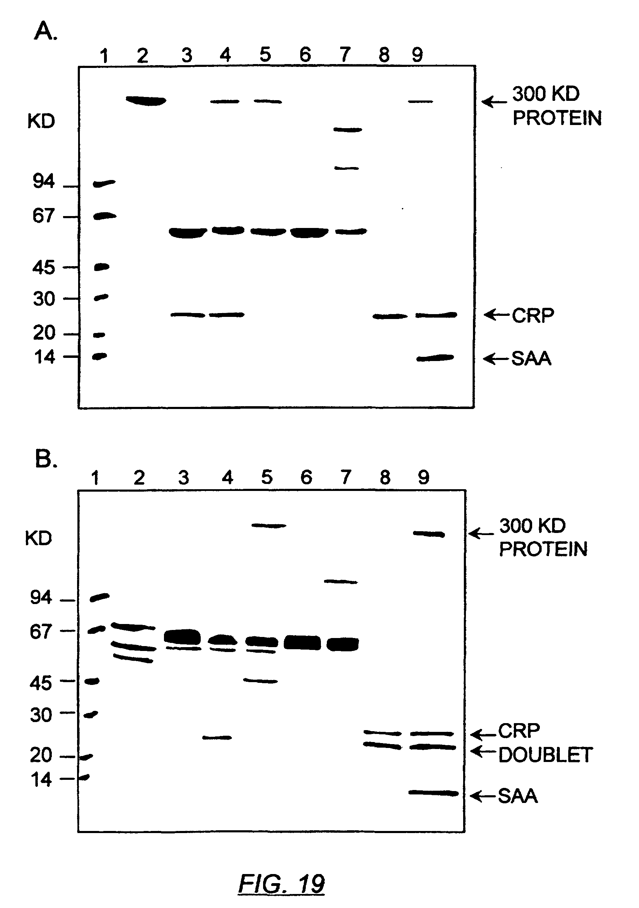

**Figures 19A** and **19B** show the non-reduced and reduced, respectively, SDS-PAGE of various fractions obtained upon fractionation of patient plasma. The loading orientation (left to right): 5-15% gradient/Neville Gel. (approximately 10µg protein loaded per well). In lane 1 are molecular weight standards (94, 67, 45, 30, 20 and 14 kDa from top to bottom. In lane 2 is 35% (NH₄)₂SO₄ pellet, whereas in lane 3 is 70% (NH₄)₂SO₄ supernate. Lane 4 is Q-sepharose starting material. Also shown in **Figures 19A** and **19B** are (from **Figure 18**) peaks 1, 2a, 2b and 3 in, respectively, lanes 5, 6, 7 and 8. Lane 9 is pellet 1, whereas in lane 10 are again, molecular weight standards.
Results of NH₂-terminal sequencing showed peak 3, the 22 kDa protein in lanes 8 and 9 to be C-reactive protein (CRP), and the 10 kDa protein in lane 9 to be human serum amyloid A (SAA). Peak 1 in lane 5 is a >300 kDa protein which, as can be seen in Figure 21, is part of the complex of proteins (along with CRP) in the precipitate formed due to the addition of a metal divalent cation to a plasma sample.

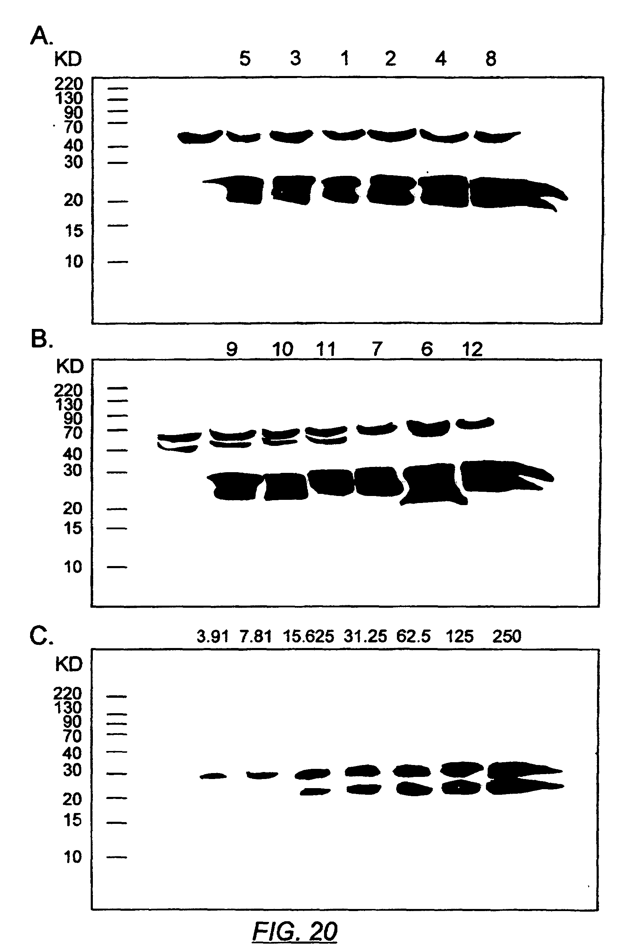

Immunoblots of CRP were performed in normal (NHP) and DIC plasma. Blot A (see **Figure 20**): (used 0.2 µl plasmas for reducing SDS-PAGE/CRP Immunoblotting). Loading orientation (left to right): NHP; Pt 5; 3; 1; 2; 4; and 8. For Blot B: Loading orientation (left to right): NHP; Pt 9; 10; 11; 7; 6; 12. For Blot C: (CRP purified from DIC patient plasma) - Loading orientation (left to right; ng CRP loaded): 3.91; 7.81; 15.625; 31.25; 62.5; 125; 250. The Blots were blocked with 2% (w/v) BSA in PBS, pH 7.4 and then sequentially probed with rabbit anti-human CRP-IgG (Sigma, Cat# C3527, dil 1:5000 in PBS/0.01% Tween 20) and then treated with the test detecting antibody conjugated to HRP (dil 1:25000 in PBS/0.01% Tween 20).

**Figure 21** illustrates the turbidity changes upon adding divalent calcium to materials obtained upon Q-sepharose chromatography in the absence of plasma. No single peak gave a positive response, but a mixture of peak 1 and peak 3 materials did give a positive response indicating the involvement of CRP, a 300 kDa protein, and one or more other proteins in the precipitate (peak 3 + plasma was the control). **Table 7** is a table shows CRP amounts in µg/ml as determined by ELISA. Delta A405nm is the maximum turbidity change observed when patients' plasmas were recalcified on the presence of the thrombin inhibitor PPACK). **Table 7**, therefore, shows that patients with increased absorbance have varying elevated levels of CRP, once again indicating that more than one protein is involved in the precipitate formation.

**TABLE 7**

| Plasma Sample | [CRP], µg/mL | Δ 405 nm |
|---|---|---|
| Normal Human Pool | 0.73 | 0 |
| Pt #1 | 248 | 0.329 |
| Pt #2 | 277 | 0.235 |
| Pt #3 | 319 | 0.345 |
| Pt #4 | 443 | 0.170 |
| Pt #5 | 478 | 0.640 |
| Pt #6 | 492 | 0.230 |
| Pt #7 | 528 | 0.140 |
| Pt #8 | 576 | 0.640 |
| Pt #9 | 600 | 0.390 |
| Pt #10 | 639 | 0.160 |

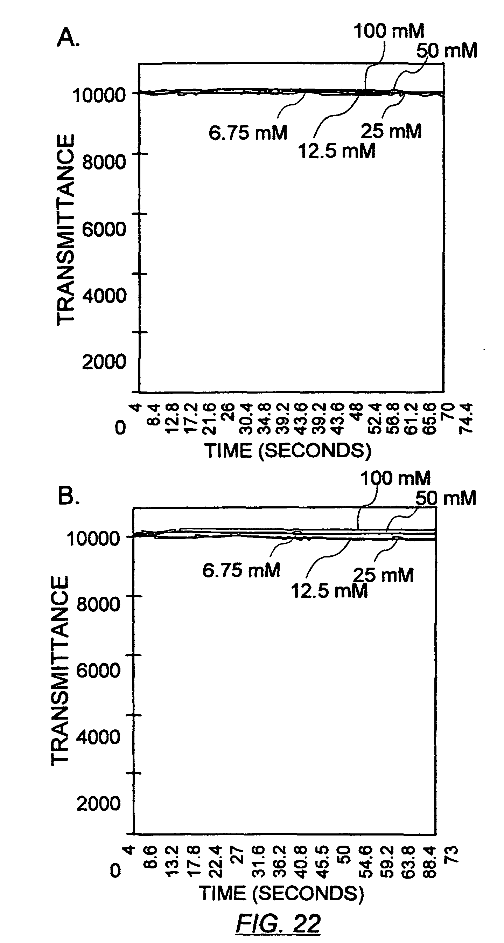

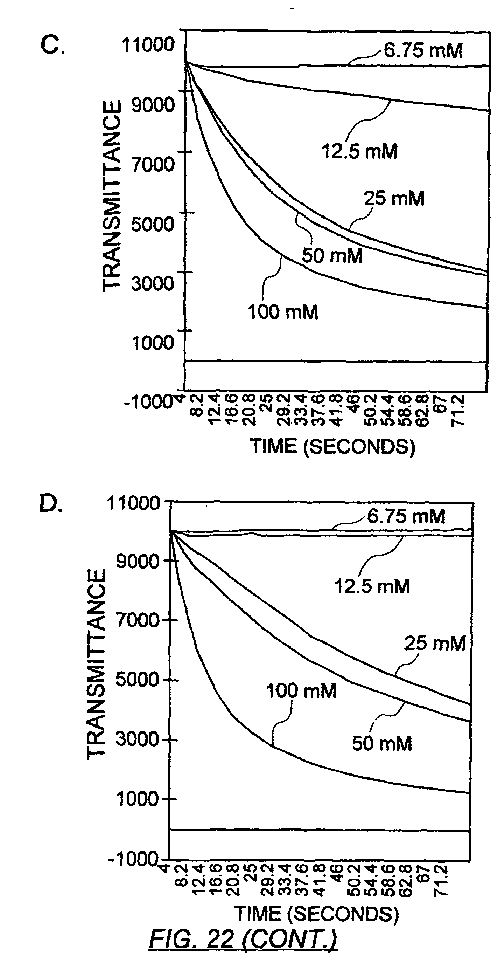

In one embodiment of the invention, the reagent to plasma ratio is varied between multiple tests using a reagent that induces precipitate formation. This variance allows for amplifying the detection of the precipitate formation by optimization of reagent to plasma ratio (e.g. varying plasma or reagent concentrations). In the alternative, the slope due to the precipitate formation can be averaged between the multiple tests. As can be seen in **Figure 22**, the response to increasing calcium concentrations is shown in optical transmission waveform profiles. Panels A and B show two normal patients where calcium concentrations were varied (no clotting agents used), whereas the panels C and D show two patients with haemostatic dysfuntion (DIC in these two cases) where the metal cation (calcium) concentration was varied (the calcium alone being incapable of any substantial fibrin polymerization).

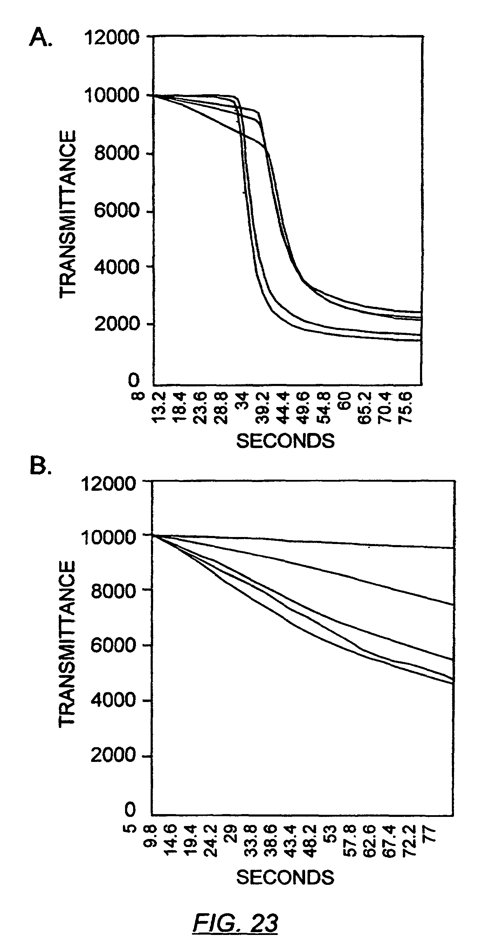

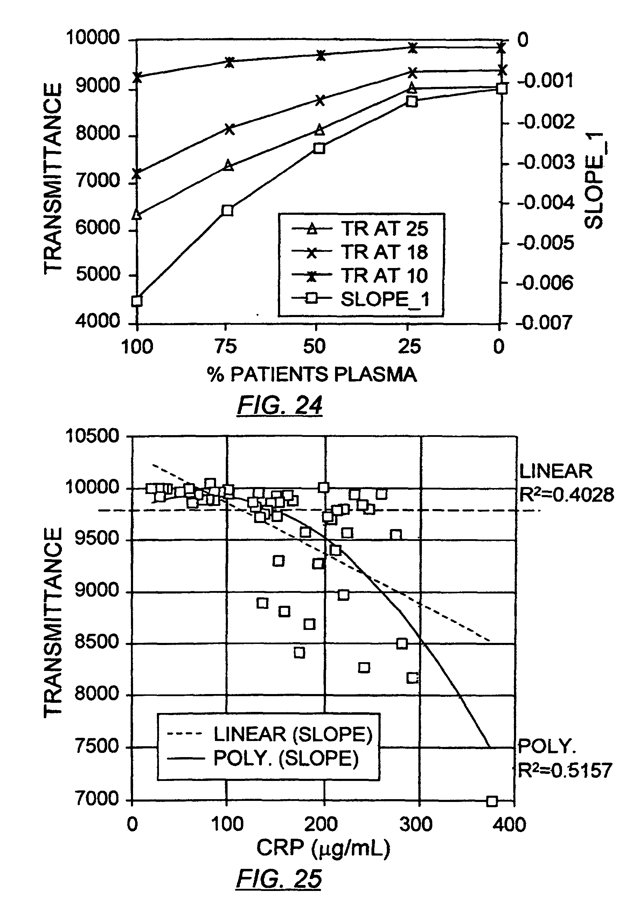

Though precipitate formation is capable of being detected in patients with haemostatic dysfunction when a clotting agent is used, it is beneficial that the reagent used is capable of forming the precipitate without fibrin polymerization. As can be seen in **Figure 23**, the slope is more pronounced and more easily detectable when a reagent such as calcium chloride is used alone (panel A) as compared to when it is used along with a clotting reagent such as an APTT reagent (panel B). As can be seen in **Figure 24**, when a clot inhibitor was added (in this case heparin), all parameters including slope_1 gave good results, and slope_1 showed the best sensitivity. For the above reasons, a reagent capable of precipitate formation in the absence of fibrin polymerization and/or a clot inhibitor are preferred.

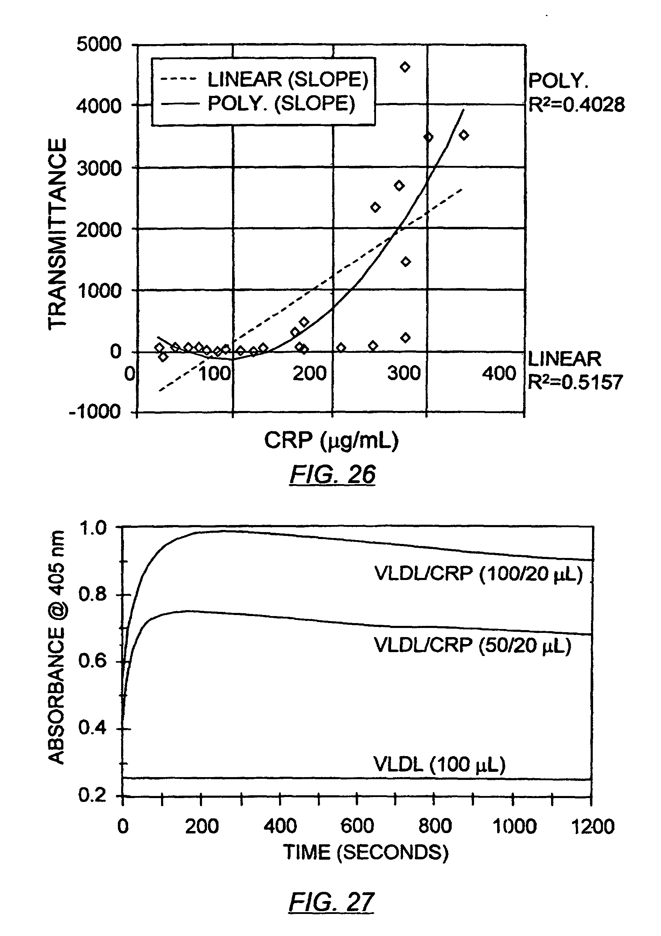

As can be seen in **Figure 25**, CRP levels from 56 ITU patients were plotted against transmittance at 18 seconds. The dotted line is the cut-off for an abnormal transmittance at 18 seconds. **Figure 26** shows more samples with CRP and decrease in transmittance at 18 seconds (10000 - TR18). These figures indicate that patients with abnormal transmittance levels due to precipitate formation all have increased levels of CRP. However, not all patients with increased levels of CRP have abnormal transmittance levels thus indicating that more than CRP is involved in the precipitate.

In a further embodiment of the invention, the formation of the precipitate comprising a complex of proteins including CRP is detected and/or quantitated, by the use of a latex agglutination assay. In this method, antibodies are raised against either the 300 kDa protein or CRP. Whether monoclonal or polyclonal antibodies are used, they are bound to suitable latex and reacted with a patient test sample or preferably with the precipitate itself having been separated from the rest of the patient plasma, in accordance with known methods. The amount of agglutination of the latex is proportional to the amount of the CRP complex in the sample.

Alternatively, immunoassays can be performed, such as ELISA's, according to known methods (sandwich, competition or other ELISA) in which the existence and/or amount of the complex of proteins is determined. For example, an antibody bound to solid phase binds to CRP in the CRP protein complex. Then, a second labeled antibody is added which also binds to CRP in the CRP protein complex, thus detecting the complex of proteins. In the alternative, the second labeled antibody can be specific for the 300 kDa protein in the complex. Or, in a different assay, the antibody bound to solid phase can bind to the 300 kDa protein in the complex, with the second (labeled) antibody binding either to the 300 kDa protein or to CRP. Such immunoassays could likewise be adapted to be specific for SAA. The above techniques are well known to those of ordinary skill in the art and are outlined in Antibodies, A Laboratory Manual, Harlow, Ed and Lane, David, Cold Spring Harbor Laboratory, 1988, the subject matter of which is incorporated herein by reference.

After further studies, it has been determined that the "300 kDa" protein is in fact the Apo(B)-100 compound of VLDL (very low density lipoprotein) having a molecular weight of from 500 to 550 kDa. There can be additional lipoprotein complexes in the precipitate as well, including CRP-LDL (CRP complexed with low density lipoprotein), CRP-IDL (CRP complexed with intermediate density lipoprotein), CRP-chylomicrons, CRP-HDL (CRP complexed with high density lipoprotein) and SAA-VLDL (serum amyloid A complexed with VLDL).

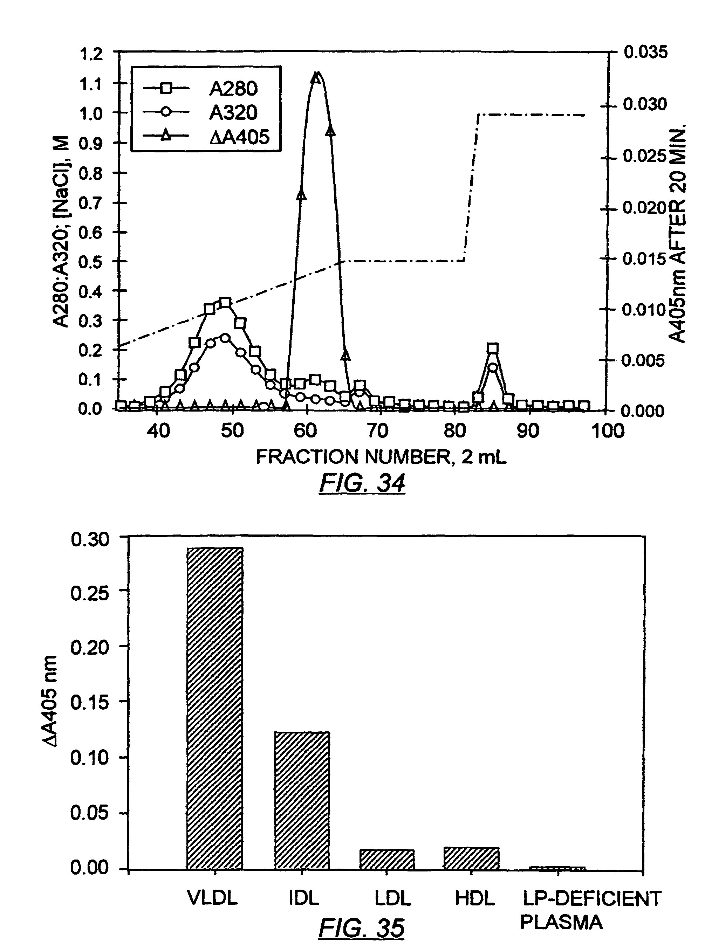

In order to characterize the components of the complex, the precipitate was dispersed in citrate and subjected to anion exchange chromatography (see **Figure 34**). The procedure yielded two major peaks (referred to hereinafter as "peak 1" and "peak 3"), the first of which was very turbid. The turbidity was obvious to the eye and was quantified by absorbance measurements at 320 nm. Fractions were tested for activity (turbidity formation in normal plasma upon recalcification). Only peak 3 exhibited turbidity when added to normal plasma.

In order to further characterize the precipitated material, lipid and protein analyses were performed. In addition, fractions obtained after anion exchange chromatography were subjected to SDS-PAGE, immunoblotting, and amino acid sequence analysis. The isolated materials were shown to comprise proteins, phospholipids, cholesterol and triglycerides in proportions typical of very low density lipoproteins (VLDL and IDL). See **Table 8.** Fractionation by anion exchange and SDS-PAGE showed that the precipitate contains Coomassie blue staining protein bands with apparent molecular masses of 500 kDa, 22 kDa and 10 kDa. The 22 kDa protein yielded an amino terminal sequence QTDMS_KAFV (**SEQ ID NO:1**), which identified the protein as C-reactive protein. The 10 kDa protein gave two residues at each cycle in the sequenator.
They were consistent with serum amyloid A beginning with amino acids 18 and 19. The 500 kDa species did not yield a sequence, likely due to the small molar amounts of it. The high molecular weight of this band, however, was consistent with apo-lipoprotein B, the major protein component of VLDL.

**TABLE 8**

| **Lipoprotein class** | **Protein** | **PL** | **UC** | **CE** | **TG** |
|---|---|---|---|---|---|
| VLDL | 10% | 15% | 6% | 14% | 53% |
| IDL | 18% | 22% | 7% | 23% | 31% |
| LDL | 25% | 21% | 9% | 42% | 4% |
| PL=phospholipid, UC=unesterified cholesterol, CE=cholesteryl esters, TG=triacylglycerol. | | | | | |

After fractionation, the high molecular weight band and SAA were obtained in peak 1, and CRP was obtained in peak 3 (see **Figure 34**). Peaks 2a and 2b were seen in **Figure 18** but not **Figure 34** because, in the assay run for **Figure 18**, the amount of protein and lipoprotein in the sample exceeded the capacity of the column. When the column is not overloaded as in the assay run for **Figure** **34**, peaks 2a and 2b do not appear. The precipitate and materials in peaks 1 and 3 were assessed by immunoblotting for Apo(B)-100, CRP and SAA. The results were consistent with the identification of the 500 kDa material as Apo(B)-100, the 22 kDa material as CRP, and the 10 kDa material as SAA.

The starting material, the materials in peaks 1 and 3, and a mixture of them were recalcified in the absence of plasma to determine which component or components were needed for the formation of a precipitate. The results showed that the starting material, but not isolated peak 1 or peak 3 components, formed a precipitate when recalcified. The mixture of peaks 1 and 3, however, did form a precipitate. Therefore, it can be concluded that VLDL and CRP are minimally required to form the precipitate. The procedure was repeated with at least 10 different positive plasmas and the results were the same. Occasionally, however, SAA was not recovered in the isolated peaks. Nonetheless, precipitates formed with VLDL and CRP in the absence of SAA. It is therefore concluded that SAA can be included in the precipitate/complex, but is not necessary for its formation.

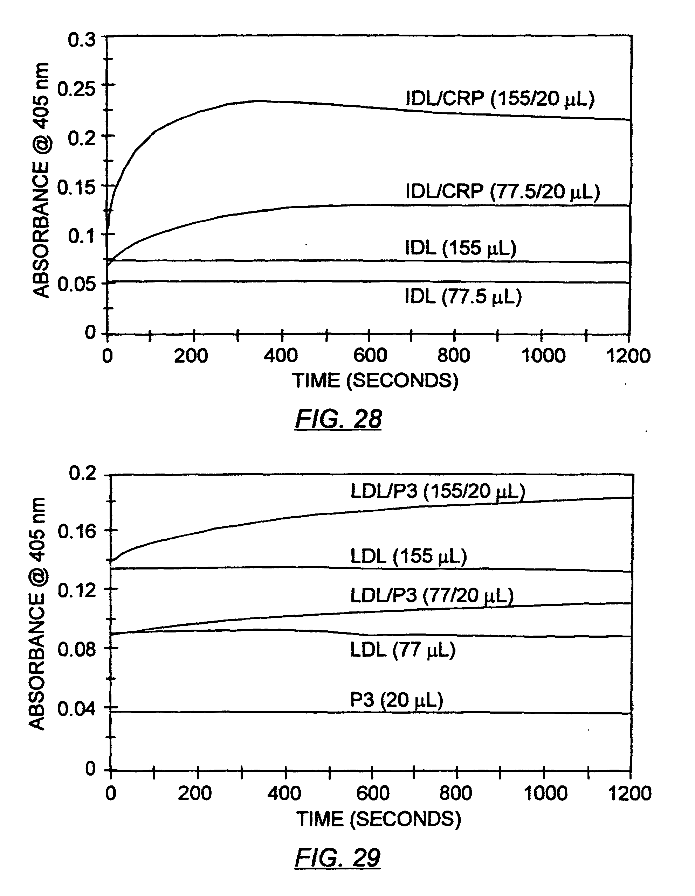

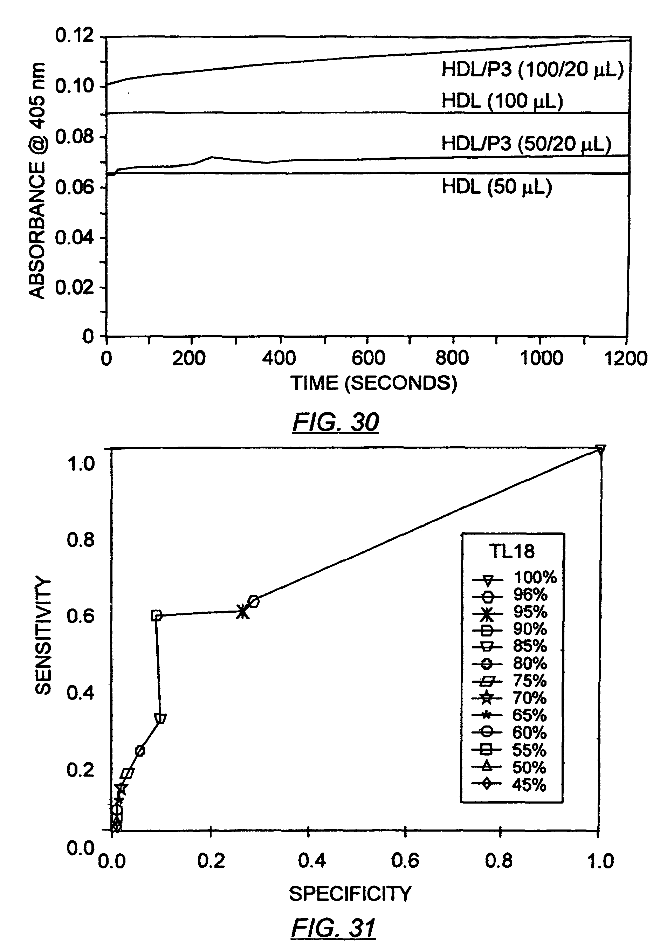

Reconstitution experiments were run to verify the ability of the above-mentioned complexes to form. As can be seen in **Figure 27,** VLDL and P3 (Peak 3 = CRP, see **Figure 18**) at varying concentrations (100/20 µl: VLDL/CRP and 50/20 µl VLDL/CRP) shows an increase in absorbance due to turbidity, in comparison with VLDL alone. Likewise, as can be seen in Figures 28 and 29, IDL and CRP, as well as LDL and CRP (and to a lesser extent HDL and CRP as can be seen in Figure 30) also cause an increase in turbidity when combined together. And, as can be further seen in **Table 9**, the different lipoproteins have different calcium-dependent turbidity activity in the presence of purified CRP.

**TABLE 9**

| **Sample** | **Total Vol Isolated (µL)** | **[Protein] (mg/mL)** | **Excursion (ΔA405 nm/µL)** | **Total Protein (mg)** | **Total Excursion (ΔA405 nm/µL)** |
|---|---|---|---|---|---|
| VLDL | 900 | 0.326 | 0.0096 | 0.29 | 8.64 |
| IDL | 2000 | 0.068 | 0.0018 | 0.136 | 3.60 |
| LDL | 1500 | 0.354 | 0.00033 | 0.531 | 0.50 |
| HDL | 2000 | 1.564 | 0.00028 | 3.13 | 0.56 |

Interestingly, it has been found that the turbidity caused when adding a divalent metal cation such as calcium to patient plasmas which exhibit the characteristic slope (even in the absence of clot formation) due to the above-noted complexes, does not correlate with the level of CRP in the patient plasma. Therefore, the present invention is not directed to detecting CRP levels per se, but rather detecting CRP complexed with lipoproteins (VLDL in particular). In the present invention, it is believed that the formation of the complex ex vivo (after adding a divalent metal cation to citrated plasma) corresponds to the existence of the complex in vivo, which is possibly an indication of the inability of that patient to clear the formed complex(es). Clearance of VLDL and IDL from the plasma by the liver is directed by their surface apo E. Therefore, if there is defective clearance of the complex(es) from the plasma, it may be due to a mutated, fragmented or otherwise defective apo E, or to an oxidized, mutated or fragmented lipoprotein (e.g. beta-VLDL, an oxidized LDL, an abnormal LDL called Lp(a), or an otherwise abnormal version of VLDL, LDL or IDL). IDL, LDL, Lp(a) and VLDL all have Apo(B)-100, which, if abnormal, may play a roll in the improper clearance of the complex(es) from the plasma. Of course a mutated, fragmented or otherwise abnormal form of CRP could also play a role in improper clearance of the complex from plasma, resulting in the characteristic slope in the clot waveform. As can be seen in **Table 10,** the change in absorbance due to complex formation does not correlate with the amount of CRP in the patient sample. The level of CRP is not generally limiting in complex formation. In fact, it was found that patients can have elevated levels of CRP and yet their plasmas do not exhibit the waveform slope mentioned herein-above. Adding additional VLDL, however, will cause those samples to undergo a turbidity change (in the presence of certain divalent metal cations such as calcium, of course).

**TABLE 10**

| **Plasma Sample** | **[CRP] µg/mL** | **Change at A405 nm with 0.05U PP** |
|---|---|---|
| Normal Human Pooled Plasma | 3.24 | 0 |
| Pt #1 | 204.08 | 0.359 |
| Pt #2 | 273.34 | 0.230 |
| Pt #3 | 331.47 | 0.609 |
| Pt #4 | 333.77 | 0.181 |
| Pt #5 | 355.48 | 0.129 |
| Pt #6 | 361.81 | 0.122 |
| Pt #7 | 389.53 | 0.308 |
| Pt #8 | 438.56 | 0.531 |
| Pt #9 | 443.62 | 0.137 |

It has also been found that the detection of precipitate formation correlates to clinical outcome, specifically patient death. Of 529 admissions to an intensive care unit, there were 178 deaths (34% baseline probability of death). The positive predictive value of death increased to 50% when patients had transmittance readings at 18 seconds of 96%, or a slope of -0.00075 or less. This predictive power increased to 77% when transmittance readings at 18 seconds were less than 65% (slope of -0.00432 or less). Using receiver operator characteristics analysis, the optimum level that maximized predictivity without compromising sensitivity was transmittance at 18 seconds cut-off value of 90% (or slope cut-off value of -0.00132 or less). The predictive value of death at this cut-off was found to be 75%. Additional data is shown in **Table 11,** where, for patient populations of 10 or more, the positive predictive value generally increases as the negative slope value or transmittance decreases. Thus, not only is the existence of the slope or decreased transmittance a predictor of future clinical outcome (e.g. likelihood of death), but in addition, the greater the formation of the precipitate (the greater the decrease in transmittance or increase in slope), the greater the predictor of the impending death. Figure 31 shows a ROC plot of sensitivity vs. specificity.

**TABLE 11**

| **TL 18** ≤ **(%)** | **Slope_1 ≥** | **Total No.** **Patients** | **Total No.** **Deaths** | **PPV (%)** |
|---|---|---|---|---|
| 96 | -0.00075 | 209 | 106 | 51 |
| 95 | -0.00078 | 195 | 101 | 52 |
| 90 | -0.00132 | 131 | 99 | 75 |
| 85 | -0.00184 | 84 | 49 | 58 |
| 80 | -0.00265 | 56 | 35 | 62 |
| 75 | -0.00315 | 35 | 25 | 71 |
| 70 | -0.00370 | 26 | 19 | 73 |
| 65 | -0.00432 | 18 | 14 | 78 |
| 60 | -0.00490 | 12 | 9 | 75 |

Data suggests that 25% of intensive care unit admissions will have a transmittance value at 18 seconds of 90% or less (slope -0.00132 or less) during their clinical course. Thus, the detection of complex formation can be a useful tool in predicting which patients are likely to die (and which in these group are more likely to die than others based on having a more severe decrease in slope or transmittance, and to allow for aggressive intervention with the hopes of preventing the (likely) impending death. The monitoring of the slope is also a way for monitoring the effects of the intervention.

Therefore, in one embodiment of the invention, the likelihood of system failure or mortality of a patient (e.g. in an intensive care setting) is determined by adding one or more reagents to a test sample from a patient comprising at least a component of a blood sample in order to cause formation of a precipitate comprising an acute phase protein and a lipoprotein. Then, the formation of the precipitate is measured, followed by correlating the formation of the precipitate formation to the likelihood of system failure or mortality of the patient. The method can be performed multiple times (e.g. daily, weekly, etc.) in order to monitor the effectiveness of a patient's therapy. The predictive value of this method alone or in combination with other medical indicators is clearly better than the predictive value without the test. The method also includes measuring the formation of the precipitate over time, such as with an automated analyzer using optical transmittance and/or absorbance. And, the amount of precipitate detected over time (or as a final endpoint) can be correlated to the probability of mortality (the greater the precipitate formation, the greater the likelihood of system failure or mortality, and vice versa). Also, the precipitate formation in this embodiment can form even in the absence of fibrin polymerization.

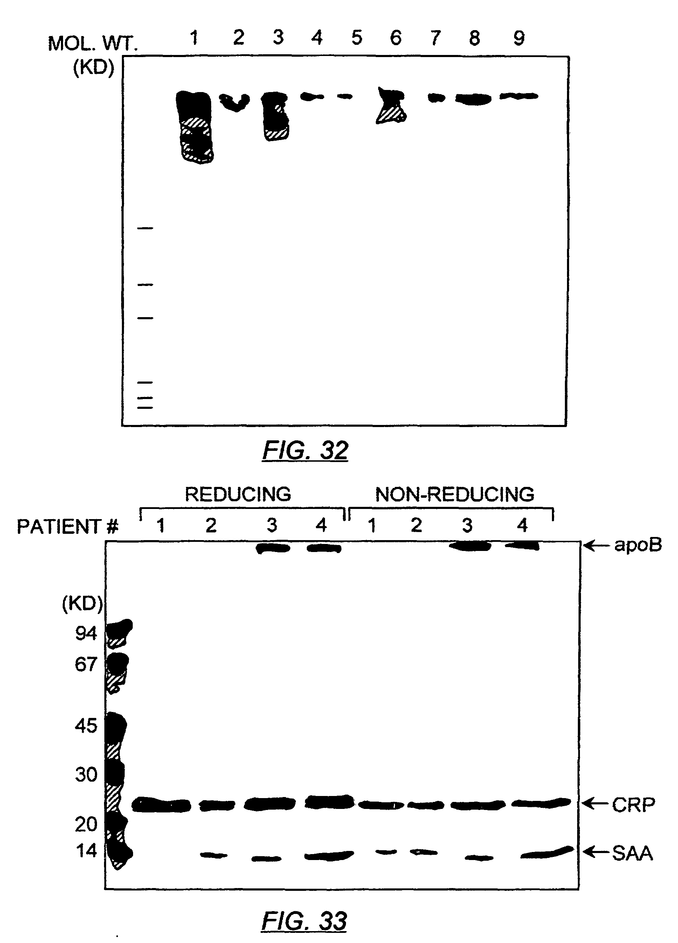

**Figure 32** is a western blot and **Figure 33** is an SDS-PAGE gel of calcium precipitates isolated from DIC patients. **Figure 32** is a western blot of a 2.5-5% SDS-PAGE gel transferred and probed with a monoclonal antibody to apoB (present on VLDL, IDL and LDL). Lane 1 in Figure 32 is normal human plasma, lanes 2-5 are DIC patient plasma, whereas lanes 6-9 are calcium precipitates from DIC patient plasmas isolated from patients studied in lanes 2-5, respectively. **Figure 33** is an 5-15% SDS-PAGE of calcium precipitates from four DIC patients electrophoresed under reducing (lanes 1-4) and non-reducing (lanes 5-8) conditions. Approximately 5 micrograms of protein was loaded from patient #1 (lanes 1,5); patient #2 (lanes 2,6); patient #3 (lanes 3,7) and patient #4 (lanes 4,8). After electrophoresis, the gel was stained in Coomassie Blue, destained and dried. CRP and SAA were identified by immunoblotting and apoB was identified by N-terminal sequencing and immunoblotting.

It was also found that the complex formation can be inhibited by phosphorylcholine, or phosphorylcholine with varying fatty acid side chains (e.g. phosphotidylcholine) or vesicles containing phosphorylcholine, phosphorylethanolamine, or phosphylethanolamine with varying fatty acid side chains (e.g. phosphotidylethanolamine) or vesicles containing phosphorylethanolamine, or EACA and the like. It is known that CRP binds directly to PC and that PC competes with lipoproteins for binding to CRP. Phosphotidylcholine was found to be a major phospholipid component in the complex.

PE, apo(A) and sphingomyelin were found to be minor components. It was also found that apo(B) can bind directly to CRP, however this is unlikely to occur in vivo (and thus is not likely to be contributing to complex formation) because apo(B) does not appear in plasma in a "free" form unattached to a lipoprotein.

Therefore, in a still further embodiment of the invention, a method is provided which includes adding one or more reagents (which may or may not cause coagulation) to a test sample from a patient in order to cause formation of a precipitate comprising an acute phase protein bound to a lipoprotein. Then, the binding of the acute phase protein to the lipoprotein is measured (either over time or as an endpoint). An inhibiting reagent is added before or after the complex-inducing reagent(s), which inhibiting reagent inhibits at least in part, the binding of the acute phase protein to the lipoprotein. The extent of inhibition is then determined (e.g. based on the amount of complex formed or not). The inhibiting reagent can be added after all or substantially all of the lipoprotein has become bound to the acute phase protein, or, the inhibiting reagent can be added even prior to adding the complex inducing reagent(s) (e.g. metal divalent cation such as calcium). The types of complex-inhibiting substances can be those such as mentioned above, or an apo-lipoprotein that binds to CRP such as apoB or apoE, or EDTA, sodium citrate, or antibodies to epitopes involved in complex formation. The complex-inhibiting reagent should preferably inhibit, as an example, CRP bound to a chylomicron or chylomicron remnant, or LDL, VLDL or IDL. The method can be performed whereby the complex-causing reagent and/or the complex-inhibiting reagent are added at more than one concentration. This embodiment can be utilized to quantitate the amount of complex and/or establish the specificity of the complex. Due to the correlation of poor clinical outcome and complex formation, in one embodiment, the complex-inhibiting reagent can be used as a therapeutic to decrease the amount of complex in vivo.

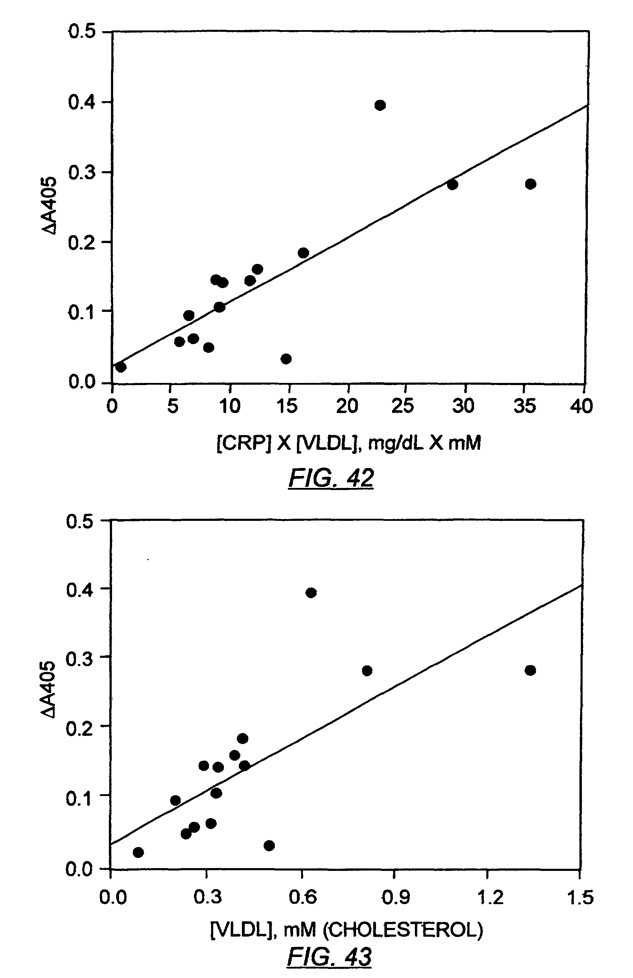

Though the primary invention is directed to detecting the complex and thereby predicting mortality, the invention is also directed to detecting total lipoprotein(s) that bind to CRP (and thus determining a total amount of certain lipoproteins in the sample). More specifically, an acute phase protein (such as CRP) is added to a test sample along with precipitate induces such as a divalent metal cation or a reagent to lower the pH at least below 7. The exogenous acute phase protein ensures that substantially all of the lipoprotein VLDL, as well as a majority of the LDL in the test sample, will form the complex/precipitate. Because the complex formation is much greater between CRP and VLDL and IDL, as compared to between CRP and LDL and HDL (see Fig. 42), in this embodiment, the complex formed by adding exogenous CRP can be correlated to total VLDL and/or VLDL + IDL levels. When adding additional CRP, the CRP can be isolated or purified CRP or recombinant CRP.

It should be understood that the present invention is useful for detecting complex formation in the absence of adding exogenous lipids to the test sample, or in the absence of adding exogenous lipids to the patient (e.g. intravenous administration of lipids such as Intralipid). Rather, the present invention is desirable for detecting a patient's own lipoproteins such as VLDL complexed with the patient's own acute phase protein(s) such as CRP. By measuring this "natural" lipoprotein-acute phase protein complex (rather than artificially causing the complex to form due to the addition of exogenous lipids), the test can be a helpful predictor of clinical outcome.

In a further embodiment of the invention the slope of the clot profile and/or the overall change in turbidity (e.g. as measured by optical transmittance or absorbance) can be utilized to diagnose the condition of the patient. More particularly, one or more reagents are added to a test sample from a patient. The test sample should include at least a component of blood from the patient (e.g. plasma or serum could be used). The reagents are capable of causing the formation of the complex in vitro, which complex comprises at least one acute phase protein and at least one lipoprotein, while causing substantially no fibrin polymerization. The formation of the complex is measured over time so as to derive a time-dependent measurement profile. Then the slope and/or overall change in turbidity ("delta") are used to diagnose the condition of the patient (e.g. predict the likelihood of mortality of the patient).

In a still further embodiment of the invention, a method for testing therapeutics (or "test compound") or treatment agents includes providing a human or animal subject whose blood undergoes complex formation and administering a therapeutic to the human or animal subject whose blood shows evidence of complex formation. Then, a therapeutic is either administered to the subject or added to the test sample *in vitro,* followed by determining whether complex formation is increased, decreased or prevented entirely. If the therapeutic is administered to the patient, it is preferable that it be administered over time and that the complex formation (or lack thereof) be likewise monitored over time.

For the purposes of the foregoing, the terms "test compound" and "therapeutic" refer to an organic compound, drug, or pharmaceutically active agent, particularly one being tested to confirm effectiveness in a clinical trial on a human or animal (preferably mammalian such as dog, cat or rat) subject (rather than an approved therapeutic agent being used to treat a disease in a particular subject). The therapeutic may, in general, be an antibiotic agent, an anti-inflammatory agent, an anticoagulant agent, a pro-coagulant agent, etc. In addition to clinical trial or drug testing use, the method may also be used in conjunction with an approved therapeutic agent such as those described above to monitor the effectiveness of the therapeutic agent in a particular patient. Thus, if the particular therapeutic is early on discovered to be ineffective for a particular patient, an opportunity is provided to switch the patient to a different therapeutic which may prove to be more effective for that patient.

**Table 12** shows CRP, VLDL, Slope 1 and the turbidity changes in 15 patients.

**Table 12**

| **Patient** **#** | **Turbidity** **(ΔA405 nm)** | **Slope _1** **X10**^{**5**} | **CRP** **(µg/mL)** | **VLDL** **Cholesterol** **(mM)** | **VLDL** **Apo** **(B)** **(mM)** | **VLDL** **Total** **Protein** **(µg/mL)** |
|---|---|---|---|---|---|---|
| 1 | 0.290 | 185 | 266 | 1.320 | 367.0 | 553.0 |
| 2 | 0.145 | 294 | 398 | 0.360 | 87.1 | 83.1 |
| 3 | 0.062 | 160 | 219 | 0.440 | 64.2 | 114.0 |
| 4 | 0.048 | 198 | 342 | 0.297 | 64.8 | 78.5 |
| 5 | 0.033 | 221 | 294 | 0.568 | 143.0 | 169.0 |
| 6 | 0.095 | 274 | 323 | 0.276 | 50.8 | 62.6 |
| 7 | 0.288 | 361 | 355 | 0.850 | 230.0 | 310.0 |
| 8 | 0.162 | 292 | 314 | 0.478 | 94.5 | 144.0 |
| 9 | 0.401 | 564 | 361 | 0.810 | 134.0 | 243.0 |
| 10 | 0.057 | 240 | 220 | 0.329 | 72.2 | 79.0 |
| 11 | 0.187 | 389 | 387 | 0.460 | 113.0 | 155.0 |
| 12 | 0.143 | 206 | 274 | 0.378 | 72.5 | 157.0 |
| 13 | 0.146 | 314 | 212 | 0.554 | 108.0 | 134.0 |
| 14 | 0.106 | 414 | 274 | 0.350 | 104.0 | 113.0 |
| 15 | 0.021 | 109 | 77 | 0.095 | 14.4 | 41.7 |

VLDL levels were measured 3 ways: 1) Total cholesterol, 2) ELISA for Apo(B), and 3) total protein by the Bradford assay.

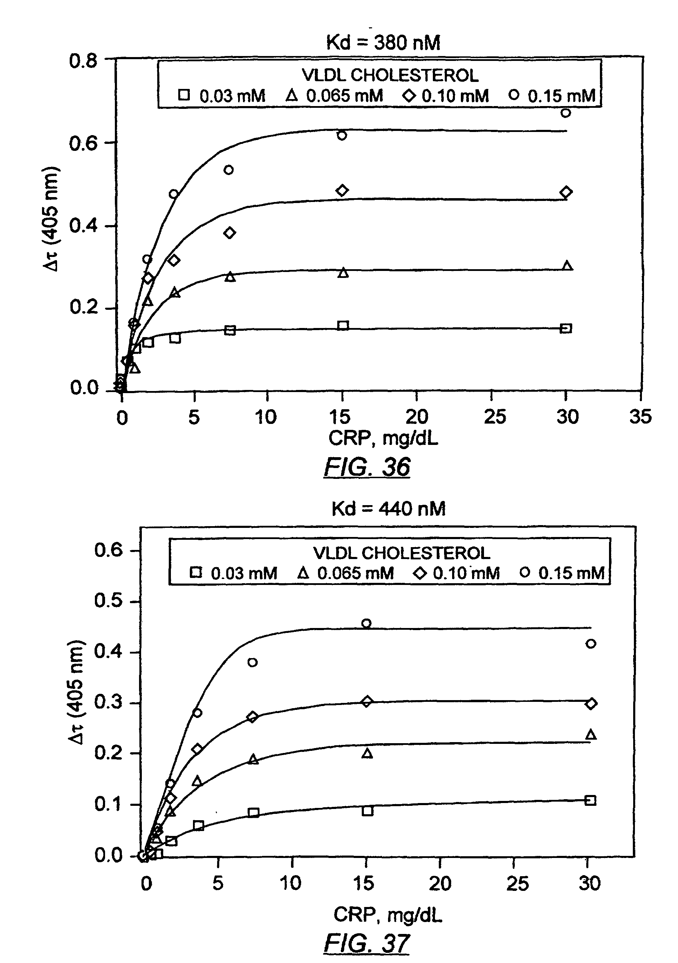

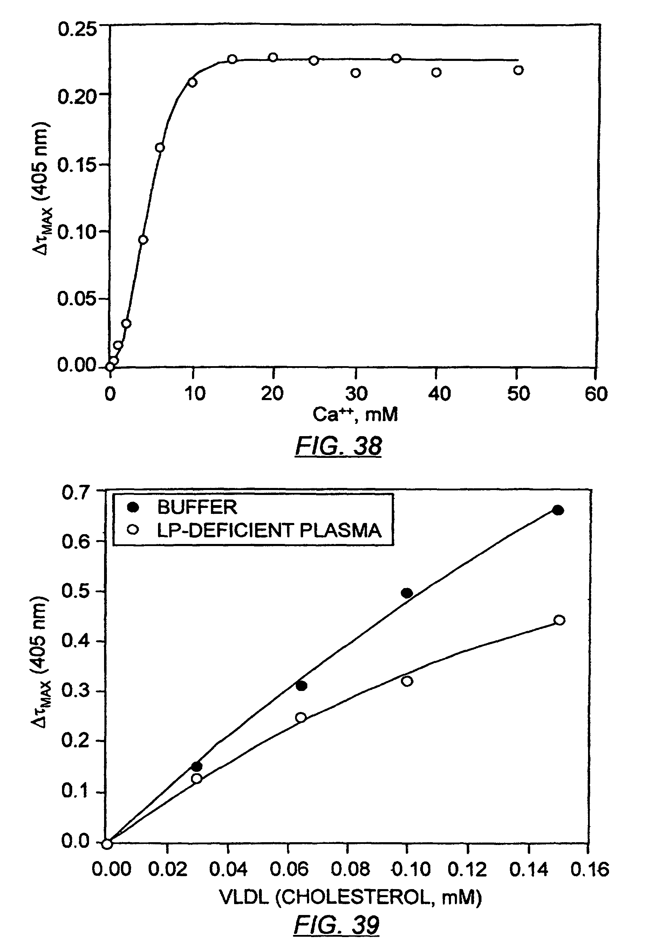

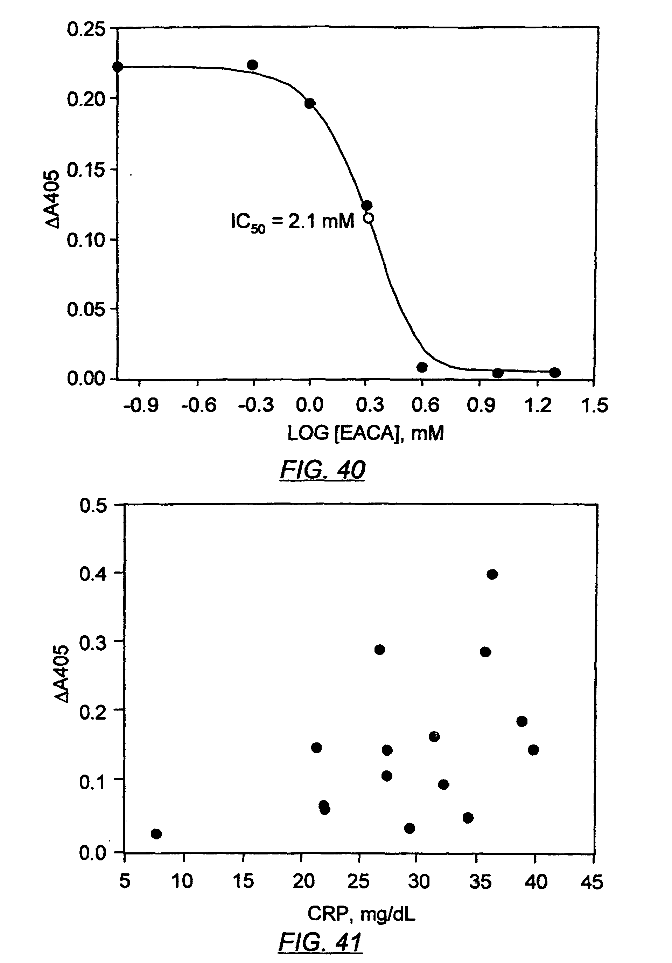

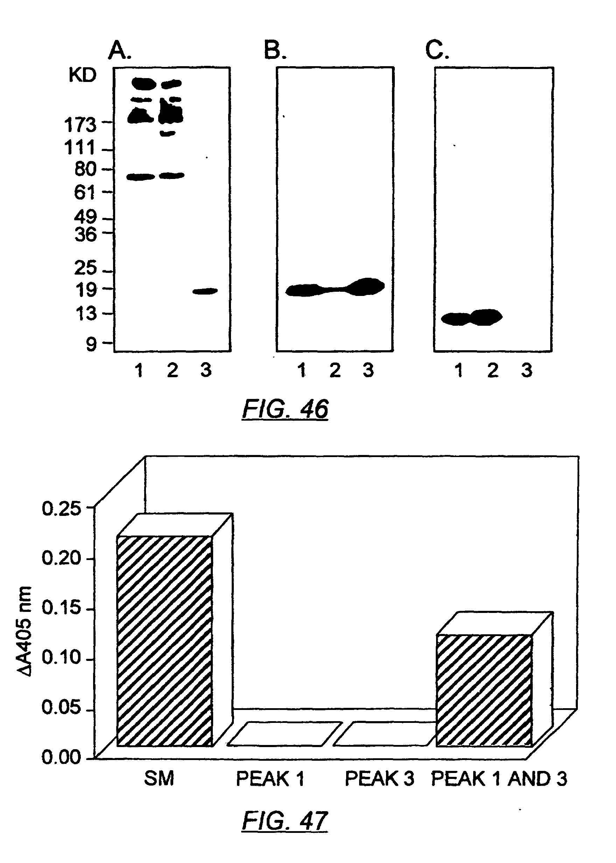

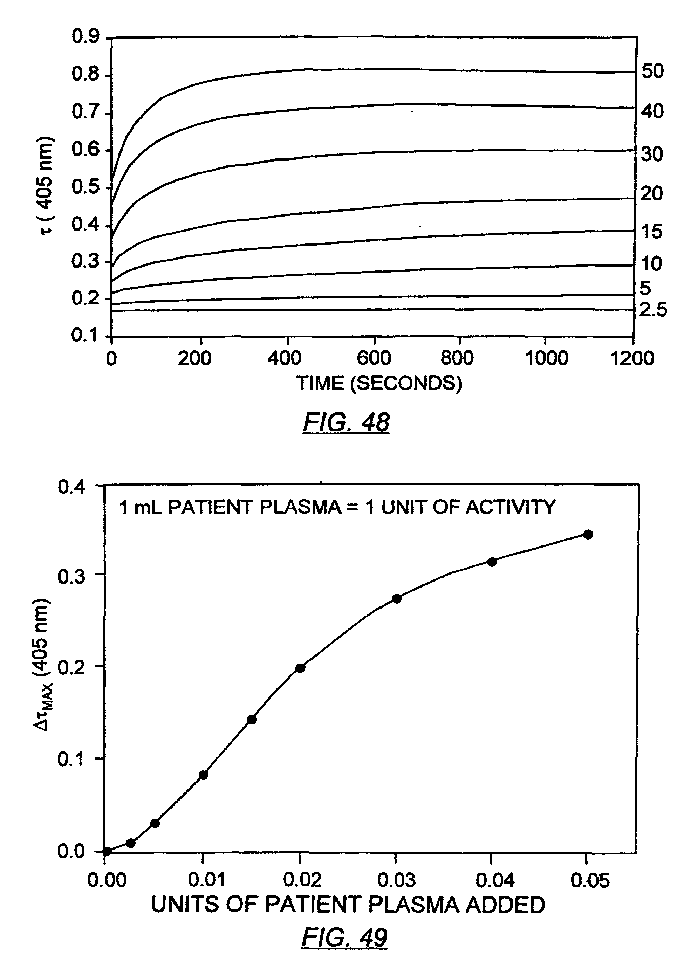

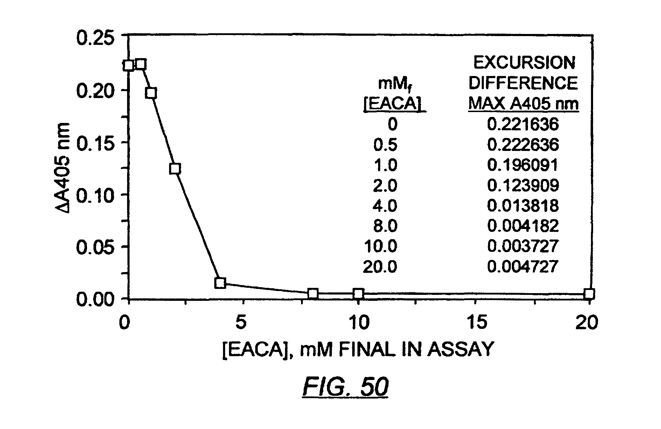

**Figures 37** through **50** illustrate further features of the present invention.

## Claims

1. A method for predicting an increased likelihood of system failure or mortality of a patient, **characterised in that** it comprises:
(a) removing a blood sample from a patient;
(b) obtaining plasma or serum from the said blood sample;
(c) adding a reagent capable of inducing the formation of a protein complex comprising at least one lipoprotein and at least one acute phase protein;
(d) taking one or more measurements of a parameter of the plasma or serum and correlating the measured parameter to complex formation if present;
and
(e) correlating the formation of the complex to an increased likelihood of system failure or mortality of the patient.

2. A method as claimed in claim 1 wherein a plurality of measurements are made after addition of the said one or more reagents in order to derive a time-dependent measurement profile.

3. A method as claimed in claim 1 wherein a single reagent is used prior to taking the said measurements.

4. A method as claimed in claim 1 wherein the said measurements are measurements of optical transmission or absorbance through the said sample, transmission being unaffected by any fibrin polymerization.

5. A method as claimed in claim 1 wherein the said reagent comprises a metal ion, preferably a divalent metal ion.

6. A method as claimed in claim 5 wherein the said metal ion comprises one or more of calcium, magnesium, manganese, iron or barium, or of a transition metal.

7. A method as claimed in claim 1 wherein a clot inhibitor is provided as part of the said one or more reagents, or as part of an additional reagent added to the said test sample.

8. A method as claimed in claim 7 wherein the said clot inhibitor comprises one or more of hirudin, heparin, PPACK, I2581 or antithrombin.

9. A method as claimed in claim 1 wherein the said one or more measurements are unaffected by clot formation due to lack of fibrin polymerization.

10. A method as claimed in claim 1 wherein the one or more measurements are a plurality of measurements, a rate of change of the said plurality of measurements or a total change being determined, and haemostatic dysfunction being determined based on the determined total and/or rate of change.

11. A method as claimed in claim 1 wherein the said at least one lipoprotein comprises VLDL, IDL and/or LDL, and the said at least one acute phase protein comprises SAA and/or CRP.

12. A method as claimed in claim 11 wherein a majority of the said complex comprises CRP bound to VLDL.

13. A method as claimed in claim 1 wherein the said reagent is capable of causing precipitate formation completely in the absence of fibrin polymerization.

14. A method as claimed in claim 1 wherein the one or more reagents is capable of inducing formation of a precipitate comprising an acute phase protein and a lipoprotein.

15. A method as claimed in claim 14 wherein it further comprises:
(i) measuring a precipitate comprising the acute phase protein and the lipoprotein;
(ii) adding an inhibiting reagent, before or after adding the said one or more precipitate-causing reagents, which inhibits at least in part the formation of the precipitate;
and
(iii)determining the extent of inhibition of the said inhibiting reagent.

16. A method as claimed in claim 15 wherein the said precipitate-inhibiting reagent is added after all or substantially all of the lipoprotein has become associated with acute phase protein so as to form the said precipitate.

17. A method as claimed in claim 15 wherein the said precipitate-inhibiting reagent is added prior to adding the precipitate-causing reagent.

18. A method as claimed in claim 14 wherein the said precipitate-inducing reagent is a divalent metal cation.

19. A method as claimed in claim 15 wherein the said precipitate-inhibiting reagent comprises one or more of an apolipoprotein capable of binding to CRP, a phosphorylcholine, EDTA, sodium citrate, or an antibody capable of binding to a lipoprotein-acute phase protein binding site.

20. A method as claimed in claim 19 wherein the said precipitate-inhibiting reagent is capable of inhibiting the association of CRP with chylomicrons or remnants thereof, LDL, VLDL and/or IDL.

21. A method as claimed in claim 20 wherein the determining of the extent of inhibition is performed over time so as to derive a time-dependent measurement profile.

22. A method as claimed in claim 21 wherein the measurement over time is a measurement of optical transmittance or absorbance over time.

23. A method as claimed in claim 1 wherein it further comprises measuring the formation of the protein complex and correlating the formation of the complex to a concentration of the said one or more lipoproteins.

24. A method as claimed in claim 23 wherein the said reagent comprises a divalent metal cation and an acute phase protein.

25. A method as claimed in claim 24 wherein the said acute phase protein is CRP.

26. A method as claimed in claim 24 wherein the said one or more lipoproteins comprises chylomicrons, VLDL and/or IDL.

27. A method as claimed in claim 24 wherein the formation of the complex and the formation of an additional complex are measured over time so as to provide respective first and second time-dependent measurement profiles.

28. A method as claimed in claim 27 wherein the measured additional complex and the measured initial complex together are correlated to a total amount of acute phase protein in the test sample.

29. A method as claimed in claim 27 wherein the acute phase protein is C-reactive protein.

30. A method as claimed in claim 29 wherein the greater the initial complex measured, the greater the likelihood of system failure and/or mortality.

31. A method as claimed in claim 1 wherein the prediction of the increased likelihood of system failure or mortality is more accurate than in the absence of performing a method for predicting an increased likelihood of system failure or mortality of a patient comprising steps (a) to (e).

32. A method as claimed in claim 1 wherein steps (a) to (e) are performed at least once more at a later time in order to determine patient condition regression or progression.

33. A method for testing the effectiveness of a therapeutic **characterised in that** it comprises:
(a) removing from a test subject a test sample to be tested for complex formation;
(b) adding to the test sample a reagent which causes formation of a complex of acute phase protein and lipoprotein present in the said test sample in the presence and absence of a therapeutic;
(c) optionally repeating steps (a) and (b);
and
(d) determining if the amount of complex formed has changed.

## Patentansprüche

1. Verfahren zur Vorhersage einer gestiegenen Wahrscheinlichkeit einer Systemstörung oder der Sterblichkeit eines Patienten, **dadurch gekennzeichnet, dass** es umfasst:
(a) Nehmen einer Blutprobe von einem Patienten;
(b) Beschaffen eines Plasmas oder Serums aus der Blutprobe;
(c) Zugeben eines Reagens, das in der Lage ist, die Bildung eines Proteinkomplexes zu induzieren, der mindestens ein Lipoprotein und mindestens ein Akute-Phase-Protein umfasst;
(d) Durchführen einer oder mehrerer Messungen eines Parameters des Plasmas oder Serums und Korrelieren des gemessenen Parameters zur Komplexbildung, wenn vorhanden; und
(e) Korrelieren der Bildung des Komplexes zu einer gestiegenen Wahrscheinlichkeit einer Systemstörung oder der Sterblichkeit des Patienten.

2. Verfahren gemäß Anspruch 1, wobei eine Vielzahl von Messungen nach der Zugabe des einen Reagens oder der mehreren Reagenzien durchgeführt werden, um ein zeitabhängiges Messprofil abzuleiten.

3. Verfahren gemäß Anspruch 1, wobei ein einzelnes Reagens vor der Durchführung der Messungen verwendet wird.

4. Verfahren gemäß Anspruch 1, wobei die Messungen Messungen der optischen Transmission oder Absorption durch die Probe sind, wobei die Transmission durch Fibrinpolymerisation nicht beeinflusst wird.

5. Verfahren gemäß Anspruch 1, wobei das Reagens ein Metallion, bevorzugt ein zweiwertiges Metallion, umfasst.

6. Verfahren gemäß Anspruch 5, wobei das Metallion eines oder mehrere aus Calcium, Magnesium, Mangan, Eisen oder Barium oder einem Übergangsmetall umfasst.

7. Verfahren gemäß Anspruch 1, wobei ein gerinnungshemmendes Mittel als Teil des einen Reagens oder der mehreren Reagenzien bereitgestellt wird, oder als Teil eines zusätzlichen Reagens zu der Testprobe zugegeben wird.

8. Verfahren gemäß Anspruch 7, wobei das gerinnungshemmende Mittel eines oder mehrere aus Hirudin, Heparin, PPACK, I2581 oder Antithrombin umfasst.

9. Verfahren gemäß Anspruch 1, wobei die eine oder die mehreren Messungen durch Gerinnungsbildung aufgrund des Fehlens von Fibrinpolymerisation unbeeinflusst sind.

10. Verfahren gemäß Anspruch 1, wobei die eine oder die mehreren Messungen eine Vielzahl von Messungen darstellen, wobei eine Änderungsrate der Vielzahl der Messungen oder durch eine Gesamtänderung bestimmt wird, und eine hämostatische Fehlfunktion, basierend auf der bestimmten Gesamtänderung und/oder Änderungsrate, bestimmt wird.

11. Verfahren gemäß Anspruch 1, wobei das mindestens eine Lipoprotein VLDL, IDL und/oder LDL und das mindestens eine Akute-Phase-Protein SAA und/oder CRP umfasst.

12. Verfahren gemäß Anspruch 11, wobei eine Mehrheit des Komplexes an VLDL gebundenes CRP umfasst.

13. Verfahren gemäß Anspruch 1, wobei das Reagens in der Lage ist, Niederschlagsbildung vollständig in der Abwesenheit von Fibrinpolymerisation auszulösen.

14. Verfahren gemäß Anspruch 1, wobei das eine Reagenz oder die mehreren Reagentien in der Lage sind, die Bildung eines Niederschlags zu induzieren, umfassend ein Akute-Phase-Protein und ein Lipoprotein.

15. Verfahren gemäß Anspruch 14, wobei es zusätzlich umfasst:
(i) Messen eines Niederschlags, der das Akute-Phase-Protein und das Lipoprotein umfasst;
(ii) Zugeben eines hemmenden Mittels vor oder nach der Zugabe des einen niederschlagsauslösenden Reagens oder der mehreren niederschlagsauslösenden Reagentien, das zumindest zum Teil die Bildung des Niederschlags hemmt; und
(iii) Bestimmen des Ausmaßes der Hemmung des hemmenden Mittels.

16. Verfahren gemäß Anspruch 15, wobei das niederschlagshemmende Mittel zugegeben wird, nachdem sich das gesamte oder im Wesentlichen das gesamte Lipoprotein mit Akute-Phase-Protein verbunden hat, um so den Niederschlag zu bilden.

17. Verfahren gemäß Anspruch 15, wobei das niederschlagshemmende Mittel vor der Zugabe des niederschlagsauslösenden Reagens zugegeben wird.

18. Verfahren gemäß Anspruch 14, wobei das niederschlagsauslösende Mittel ein zweiwertiges Metallkation ist.

19. Verfahren gemäß Anspruch 15, wobei das niederschlagshemmende Mittel eines oder mehrere von einem Apolipoproteins, das in der Lage ist, an CRP zu binden, einem Phosphorylcholin, EDTA, Natriumcitrat oder einem Antikörper, der in der Lage ist, an eine Lipoprotein-Akute-Phase-Protein Bindungsstelle zu binden, umfasst.

20. Verfahren gemäß Anspruch 19, wobei das niederschlagshemmende Mittel in der Lage ist, die Assoziation von CRP mit Chylomikronen oder Resten davon, LDL, VLDL und/oder IDL zu hemmen.

21. Verfahren gemäß Anspruch 20, wobei die Bestimmung des Ausmaßes der Hemmung über die Zeit durchgeführt wird, um so ein zeitabhängiges Messprofil abzuleiten.

22. Verfahren gemäß Anspruch 21, wobei die zeitabhängige Messung eine Messung der optischen Transmission oder Absorption über die Zeit ist.

23. Verfahren gemäß Anspruch 1, wobei es zusätzlich das Messen der Bildung des Proteinkomplexes und das Korrelieren der Bildung des Komplexes zu einer Konzentration des einen Lipoproteins oder der mehreren Lipoproteine umfasst

24. Verfahren gemäß Anspruch 23, wobei das Reagens ein zweiwertiges Metallkation und ein Akute-Phase-Protein umfasst.

25. Verfahren gemäß Anspruch 24, wobei das Akute-Phase-Protein CRP ist.

26. Verfahren gemäß Anspruch 24, wobei das eine oder die mehreren Lipoproteine Chylomikrone, VLDL und/oder IDL umfassen.

27. Verfahren gemäß Anspruch 24, wobei die Bildung des Komplexes und die Bildung eines zusätzlichen Komplexes zeitabhängig so bestimmt werden, dass ein erstes bzw. zweites zeitabhängiges Messprofil bereitgestellt werden.

28. Verfahren gemäß Anspruch 27, wobei der gemessene zusätzliche Komplex und der gemessene ursprüngliche Komplex zusammen zu einer Gesamtmenge an Akute-Phase-Protein in der Testprobe korreliert werden.

29. Verfahren gemäß Anspruch 27, wobei das Akute-Phase-Protein ein C-reaktives Protein ist.

30. Verfahren gemäß Anspruch 29, wobei die Wahrscheinlichkeit einer Systemstörung und/oder Sterblichkeit umso höher ist, je größer der anfängliche Komplex gemessen wurde.

31. Verfahren gemäß Anspruch 1, wobei die Vorhersage der gestiegenen Wahrscheinlichkeit einer Systemstörung oder Sterblichkeit genauer ist als ohne das Durchführen eines Verfahrens zum Vorhersagen einer gestiegenen Wahrscheinlichkeit einer Systemstörung oder Sterblichkeit eines Patienten, das die Schritte (a) bis (e) umfasst.

32. Verfahren gemäß Anspruch 1, wobei die Schritte (a) bis (e) mindestens einmal mehr zu einem späteren Zeitpunkt durchgeführt werden, um den Rückschritt oder Fortschritt des Zustands des Patienten zu bestimmen.

33. Verfahren zum Bestimmen der Wirksamkeit eines Therapeutikums, **dadurch gekennzeichnet, dass** es Folgendes umfasst:
(a) Entnehmen einer Testprobe aus einem Testsubjekt, das auf Komplexbildung überprüft werden soll;;
(b) Zugeben eines Reagens zu der Testprobe, das die Bildung eines Komplexes aus Akute-Phase-Protein und Lipoprotein, die in der Testprobe vorliegen, in Gegenwart und Abwesenheit eines therapeutischen Mittels hervorruft;
(c) wahlweise Wiederholen der Schritte (a) und (b); und
(d) Bestimmen, ob die Menge an gebildetem Komplex sich geändert hat.

## Revendications

1. Procédé de prédiction d'un risque accru de défaillance du système ou de mortalité d'un patient, **caractérisé en ce qu'**il comprend :
(a) le prélèvement d'un échantillon de sang sur un patient ;
(b) l'obtention de plasma ou de sérum à partir dudit échantillon de sang ;
(c) l'ajout d'un réactif capable d'induire la formation d'un complexe protéique comprenant au moins une lipoprotéine et au moins une protéine de phase aiguë ;
(d) la prise d'une ou de plusieurs mesures d'un paramètre du plasma ou du sérum et la mise en corrélation du paramètre mesuré avec la formation du complexe si elle est présente ;
et
(e) la mise en corrélation de la formation du complexe avec un risque accru de défaillance du système ou de mortalité du patient.

2. Procédé selon la revendication 1, dans lequel une pluralité de mesures sont effectuées après l'ajout desdits un ou plusieurs réactifs afin d'en déduire un profil de mesures en fonction du temps.

3. Procédé selon la revendication 1, dans lequel un réactif unique est utilisé avant la prise desdites mesures.

4. Procédé selon la revendication 1, dans lequel lesdites mesures sont des mesures de transmission optique ou d'absorbance à travers ledit échantillon, la transmission n'étant pas affectée par une polymérisation quelconque de la fibrine.

5. Procédé selon la revendication 1, dans lequel ledit réactif comprend un ion métallique, de préférence un ion métallique divalent.

6. Procédé selon la revendication 5, dans lequel ledit ion métallique comprend un ou plusieurs éléments parmi le calcium, le magnésium, le manganèse, le fer ou le baryum, ou un ou plusieurs métaux de transition.

7. Procédé selon la revendication 1, dans lequel un inhibiteur de la coagulation est prévu en tant que partie desdits un ou plusieurs réactifs, ou en tant que partie d'un réactif additionnel ajouté audit échantillon d'essai.

8. Procédé selon la revendication 7, dans lequel ledit inhibiteur de la coagulation comprend un ou plusieurs éléments parmi l'hirudine, l'héparine, la PPACK, l'I2581 ou l'antithrombine.

9. Procédé selon la revendication 1, dans lequel lesdites une ou plusieurs mesures ne sont pas affectées par la formation d'un caillot en raison de l'absence de polymérisation de la fibrine.

10. Procédé selon la revendication 1, dans lequel la ou les mesures sont une pluralité de mesures, un taux de changement de ladite pluralité de mesures ou un changement total qui est déterminé, et un dysfonctionnement hémostatique qui est déterminé sur la base du taux de changement et/ou du changement total déterminé.

11. Procédé selon la revendication 1, dans lequel ladite au moins une lipoprotéine comprend la VLDL, l'IDL et/ou la LDL, et ladite au moins une protéine de phase aiguë comprend la SAA et/ou la CRP.

12. Procédé selon la revendication 11, dans lequel une majorité dudit complexe comprend une CRP fixée à une VLDL.

13. Procédé selon la revendication 1, dans lequel ledit réactif est capable d'entraîner la formation d'un précipité complètement en l'absence de polymérisation de la fibrine.

14. Procédé selon la revendication 1, dans lequel le ou les réactifs sont capables d'induire la formation d'un précipité comprenant une protéine de phase aiguë et une lipoprotéine.

15. Procédé selon la revendication 14, comprenant en outre :
(i) la mesure d'un précipité comprenant la protéine de phase aiguë et la lipoprotéine ;
(ii) l'ajout d'un réactif inhibiteur, avant ou après l'ajout desdits un ou plusieurs réactifs entraînant un précipité, qui inhibe au moins en partie la formation du précipité ;
et
(iii) la détermination du degré d'inhibition dudit réactif inhibiteur.

16. Procédé selon la revendication 15, dans lequel ledit réactif inhibant le précipité est ajouté après que la totalité ou sensiblement la totalité de la lipoprotéine s'est associée à la protéine de phase aiguë afin de former ledit précipité.

17. Procédé selon la revendication 15, dans lequel ledit réactif inhibant le précipité est ajouté avant l'ajout du réactif entraînant le précipité.

18. Procédé selon la revendication 14, dans lequel ledit réactif induisant le précipité est un cation métallique divalent.

19. Procédé selon la revendication 15, dans lequel ledit réactif inhibant le précipité comprend un ou plusieurs éléments parmi une apolipoprotéine capable de se fixer à la CRP, une phosphorylcholine, l'EDTA, le citrate de sodium, ou un anticorps capable de se fixer à un site de liaison lipoprotéine/protéine de phase aiguë.

20. Procédé selon la revendication 19, dans lequel ledit réactif inhibant le précipité est capable d'inhiber l'association de la CRP avec des chylomicrons ou des restes de ceux-ci, la LDL, la VLDL et/ou l'IDL.

21. Procédé selon la revendication 20, dans lequel la détermination du degré d'inhibition est réalisée dans le temps afin d'en déduire un profil de mesures en fonction du temps.

22. Procédé selon la revendication 21, dans lequel la mesure dans le temps est une mesure de transmittance optique ou d'absorbance dans le temps.

23. Procédé selon la revendication 1, comprenant en outre la mesure de la formation du complexe protéique et la mise en corrélation de la formation du complexe avec une concentration desdites une ou plusieurs lipoprotéines.

24. Procédé selon la revendication 23, dans lequel ledit réactif comprend un cation métallique divalent et une protéine de phase aiguë.

25. Procédé selon la revendication 24, dans lequel ladite protéine de phase aiguë est la CRP.

26. Procédé selon la revendication 24, dans lequel lesdites une ou plusieurs lipoprotéines comprennent des chylomicrons, la VLDL et/ou l'IDL.

27. Procédé selon la revendication 24, dans lequel la formation du complexe et la formation d'un complexe additionnel sont mesurées dans le temps afin de fournir des premier et second profils de mesures en fonction du temps respectifs.

28. Procédé selon la revendication 27, dans lequel le complexe additionnel mesuré et le complexe initial mesuré sont tous deux mis en corrélation avec une quantité totale de protéine de phase aiguë dans l'échantillon d'essai.

29. Procédé selon la revendication 27, dans lequel la protéine de phase aiguë est la protéine réactive C.

30. Procédé selon la revendication 29, dans lequel plus le complexe initial mesuré est important, plus le risque de défaillance du système et/ou de mortalité est important.

31. Procédé selon la revendication 1, dans lequel la prédiction du risque accru de défaillance du système ou de mortalité est plus précise qu'en l'absence de réalisation d'un procédé de prédiction d'un risque accru de défaillance du système ou de mortalité d'un patient comprenant les étapes (a) à (e).

32. Procédé selon la revendication 1, dans lequel les étapes (a) à (e) sont effectuées au moins une fois de plus ultérieurement afin de déterminer la régression ou la progression de l'état du patient.

33. Procédé de test de l'efficacité d'un agent thérapeutique **caractérisé en ce qu'**il comprend :
(a) le prélèvement sur un sujet d'essai d'un échantillon d'essai à tester pour la formation d'un complexe ;
(b) l'ajout à l'échantillon d'essai d'un réactif qui entraîne la formation d'un complexe de protéine de phase aiguë et de lipoprotéine présent dans ledit échantillon d'essai en présence et en l'absence d'un agent thérapeutique ;
(c) la répétition éventuelle des étapes (a) et (b) ;
et
(d) le fait de déterminer si la quantité de complexe formé a changé.
